# EUROPEAN PATENT SPECIFICATION

(11) **EP 0 998 495 B1**
(45) Date of publication and mention of the grant of the patent: **27.12.2006**
(21) Application number: 98905006.7
(22) Date of filing: 05.02.1998
(51) Int. Cl.: C07K 14/47, G01N 33/68

(54) **AMYLOID BETA PROTEIN (GLOBULAR ASSEMBLY AND USES THEREOF)**
AMYLOID BETA PROTEIN (GLOBULARER AUFBAU UND SEINE VERWENDUNG)
PROTEINE AMYLOIDE BETA (ENSEMBLE GLOBULAIRE ET SES UTILISATIONS)

(30) Priority: 05.02.1997 US 796089
(43) Date of publication of application: 10.05.2000
(73) Proprietor: Northwestern University, Evanston Illinois 60208 (US); THE UNIVERSITY OF SOUTHERN CALIFORNIA, Los Angeles, California 90089 (US)
(72) Inventor: KRAFFT, Grant, A., Glenview, IL 60025 (US); KLEIN, William, L., Winnetka, IL 60093 (US); CHROMY, Brett, A., Evanston, IL 60201 (US); LAMBERT, Mary, P., Glenview, IL 60025 (US); FINCH, Caleb, E., Altadena, CA 91101 (US); MORGAN, Todd, Manhattan Beach, CA 90266 (US); WALS, Pat, Los Angeles, CA 90065 (US); ROZOVSKY, Irina, Pasadena, CA 91107 (US); BARLOW, Ann, Evanston, IL 60201 (US)
(74) Representative: Brown, John D.
(86) International application number: PCT/US1998/002426
(87) International publication number: WO 1998/033815

(56) References cited:
- WO-A1-94/10569
- Y.-M. KUO ET AL.: "Water-soluble A beta (N-40, N42) Oligomers in Normal and Alzheimer Disease Brains" JOURNAL OF BIOCHEMISTRY, vol. 271, no. 8, 23 February 1996, TOKYO JP, pages 4077-4081, XP002068752
- A.E. ROHER ET AL.: "Morphology and Toxicity of A beta-(1-42) Dimer Derived form Neuritic and Vascular Amyloid Deposits of Alzheimer Disease" JOURNAL OF BIOCHEMISTRY, vol. 271, no. 34, 23 August 1996, TOKYO JP, pages 20631-20635, XP002068753
- A.E. ROHER ET AL.: "beta-Amyloid-(1-42) is a mayor component of cerebrobascular amyloid deposits: Implications for the pathology of Alzheimer disease" PROCEEDINGS OF THE NATIONAL ACADEMY OF SCIENCES OF USA, vol. 90, November 1993, WASHINGTON US, pages 10836-10840, XP002068754
- T. WISNIEWSKI ET AL.: "Alzheimer's Disease and Soluble A beta" NEUROBIOLOGY OF AGING, vol. 15, no. 2, 1994, pages 143-152, XP002068755

## Description

### TECHNICAL FIELD OF INVENTION

The present invention pertains to a new composition of matter, amyloid beta-derived dementing ligands (ADDLs). ADDLs comprise amyloid β peptide assembled into soluble globular non-fibrillar oligomeric structures that arc capable of activating specific cellular processes. The invention also provides methods for assaying the formation, presence, receptor protein binding and cellular activities of ADDLs. Also described are compounds that block the formation or activity of ADDLs, and methods of identifying such compounds ADDL formation and activity is relevant *inter alia* to learning and memory. Modulation of ADDL formation or activity thus can be employed according to the invention in the treatment of learning and memory disorders, as well as other diseases, disorders or conditions that are due to the effects of the ADDLs.

### BACKGROUND OF THE INVENTION

Alzheimer's disease is a progressive neurodegenerative disease, characterized by distinct pathologies, including neurofibrillary tangles, neuritic plaques, neuronal atrophy, dendritic pruning and neuronal death. From a historical perspective, definitive diagnosis of Alzheimer's disease always has relied upon identification of specific pathologic hallmarks, namely the neurofibrillary tangles which represent the collapsed cytoskeleton of dead and dying neurons, and neuritic plaques, which are extracellular deposits of various protein, lipid, carbohydrate and salt compounds, the primary protein component of which is a 39-43 residue peptide known as amyloid β.

From the Standpoint of disease impact, however, it is the symptoms manifest in Alzheimer's disease, namely the loss of memory, the erosion of cognitive functions, and the significant changes in personality and behavior, which are most significant, Underlying these symptomatic changes are specific cellular mechanisms that cause nerve cells to malfunction, and eventually to degenerate and die. Thesecellular mechanisms undoubtedly operate within a background environment that variously affords some level of protection, or exerts contributing and exacerbating effects. The result is a very broad age/incidence distribution curve, with few clues from population studies that point to specific causes.

Molecular genetics represents one realm of study where a clear picture of familial Alzheimer's disease is emerging. As described in more detail below, it is now clear from studies identifying mutations in 3 different proteins, APP and the presenilins 1 and 2, that the final common pathway leading to Alzheimer's disease is the increased production of amyloid β 1-42 (as well as amyloid β 1-43), which occurs in all of these different familial AD mutations. This is particularly noteworthy, because ADDLs, the central focus of the invention described herein, only form as stable entities from this longer form of amyloid, and not from the more prevalent, shorter form Aβ 1-40.

Amyloid β in Alzheimer's Disease. In 1984, Glenner and Wong succeeded in isolating and identifying the cerebrovascular amyloid associated with Alzheimer's disease (Glenner et al., *Biochem. Biophys. Res. Commun.,* **120**, 885-890, 1984a). Subsequently, the same 39-43 residue peptides now known as amyloid β were identified as the major protein component of Alzheimer's disease neuritic plaques (Glenner et al., *Biochem. Biophys. Res. Commun.,* **122,** 1131-1135 1984b; Masters et al., *EMBO J.,* **4,** 2757-2764, 1985a; Masters et al., *Proc. Natl. Acad. Sci.,* **82,** 4245-4249, 1985b). This was the first time a discrete molecule had been linked to Alzheimer's disease, a disease which to that point had been characterized only by neuroanatomy and neuropathology descriptions. Amyloid β also was identified as the plaque component in brains of Down's Syndrome individuals, (Glenner et al, *Biochem. Biophys. Res. Commun.,* **122,** 1131-1135, 1984b; Masters et al., *EMBO J.,* **4**, 2757-2764, 1985a; Masters et al., *Proc. Natl. Acad. Sci.,* **82**, 4245-4249, 1985b) leading to the suggestion that the gene encoding it might exist on chromosome 21. By 1987, a number of groups had used the amyloid β sequence information and molecular genetics techniques to validate that suggestion, identifying the gene for the amyloid precursor protein (APP) (Kang et al., *Nature,* **325,** 733, 1987; Tanzi et al., *Science,* **235**, 880-884, 1987).

The APP gene is a large, multi-exon gene that is differentially spliced into a number of APP's (reviewed in Selkoe, *In, Annual Review of Neuroscience,* Cowan (Ed.), **17**, *ix* + 623 p, 489-517, 1994). The proteins are large transmembrane proteins, now known to be processed by several pathways, one or more of which may generate amyloid β. The earliest studies of APP processing had suggested that amyloid β formation was not a normal process (Esch et al., *Science,* **248**, 1122-1124 1990; Sisodia et al., *Science,* **248**, 492-495, 1990), though subsequent studies in cultured cells and analysis of serum and cerebrospinal fluid have shown that amyloid β formation occurs as a normal process in many cell types, though its formation may not represent a predominant overall pathway.

Pivotal genetic studies of DNA from individuals afflicted with early onset of familial Alzheimer's disease revealed that mutations in a single gene, this same APP gene, were causative for this very severe form of the disease. Interestingly, several different mutations in the APP gene were found including three different single residue substitutions at Val 717, four residues downstream of the amyloid β 1-42 C-terminus (Goate et al., *Nature,* **349**, 704-6 1991; Chartier-Harlan et al., *Nature,* **353**, 844-6 1991; Murrell et al., *Science,* **254**, 97-9, 1991), and a two residue mutation (670-671) immediately upstream of the amyloid β N-terminus, associated with early onset familial Alzheimer's disease in a Swedish family (Mullan et al., *Nature Genetics* **1**, 345-347, 1992). When a vector encoding the cDNA of the Swedish mutant APP gene was transfected into cell lines to evaluate APP processing, it was found that six-eight times more amyloid β was formed, when compared with levels from wild-type APP (Citron et al., *Nature,* **360,** 672-674, 1992; Cai et al., *Science,* **259**, 514-516, 1993). It was also demonstrated that brain tissue extracts containing native human brain protease activities were able to process a fluorogenic octapeptide substrate encompassing the Swedish mutation more than 100-fold faster than the corresponding substrate based on the wild-type sequence (Ladror et al., *J. Biol. Chem.,* **269**, 18422-8, 1994). These results suggest that the mechanism by which the Swedish mutation causes early onset familial Alzheimer's disease involves substantial overproduction of amyloid β. Similar studies of amyloid formation in cells transfected with the 717 mutant APP also had been conducted, but the levels of amyloid β produced were not different from levels produced by wild-type APP. This led to mechanistic speculations that something other than amyloid β production was pathogenic for these mutations. A closer evaluation of processing of the APP 717 mutant, and the Swedish mutant APP by Younkin and co-workers (Suzuki et al., *Science,* **264**, 1336-1340, 1994) provided a unified picture of these genetic Alzheimer's disease cases. In this study, not only were total levels of amyloid β production evaluated, but the specific lengths of the amyloid β peptides produced were also analyzed. The results confirmed that the 717 mutation led to more than a doubling of the ratio of amyloid β 1-42 to amyloid β 1-40 (a highly soluble peptide under physiologic conditions) even though total amyloid β levels did not change. The recently discovered presenilin 1 and 2 familial Alzheimer's disease mutations in genes residing on chromosome 14 (Sherrington et al., *Nature,* **375**, 754-758, 1995) and chromosome 1 (Levy-Lahad et al., *Science,* **269**, 970-973, 1995), respectively, have also been linked to significant overproduction of amyloid β 1-42. (Mann et al., *Annals of Neurology,* **40**, 149-56, 1996; Schuener et al., *Nature Medicine,* **2**, 864-70, 1996). Based on these findings, it appears that the pathogenic process mediated by these distinctly different familial Alzheimer's disease mutations is the production of greater levels of amyloid β 1-42. This is the form of amyloid that aggregates most readily (Snyder et al., *Biophys. J.,* **67,** 1216-28, 1994), that seeds aggregation of amyloid β to form neuritic plaques (Roher et al., *Neurochem.,* **61,** 1916-1926, 1993; Tamaoka et al*., Biochem. Biophs. Res. Commun.,* **205**, 834-842, 1994), and, as described herein, the form which unexpectedly forms stable higher order assemblies termed "ADDLs".

Non-amyloid Plaque Components in Alzheimer's Disease. Amyloid β is the major protein component of plaques, comprising more than 70% of the total protein. A variety of other protein components also are present, however, including α1-antichymotrypsin (ACT), heparin sulfate proteoglycans (HSPG), apolipoproteins E and J, butyrylcholinesterase (BChE), S-100B, and several complement components. While the importance of these components in the onset and progression of Alzheimer's disease has not been established, involvement of apo E isoforms in the disease has been established by genetic studies of Roses and colleagues (Strittmatter et al., *Proc. Natl. Acad. Sci. USA,* **90**, 1977-81, 1993), who discovered that a polymorphism in the apolipoprotein E gene, namely apo E4, correlated with earlier onset of Alzheimer's disease in a large set of late-onset familial Alzheimer's disease cases. Subsequent studies have confirmed that groups of individuals with apo E4 have a significantly greater risk of Alzheimer's disease and that the onset of Alzheimer's disease roughly parallels the gene dosage for apo E4. On a mechanistic level, studies have revealed that apo E4 binds with lower affinity to amyloid β than apo E3 or apo E2, isoforms which are associated with later onset of Alzheimer's disease. It has been suggested that these isoforms may exert a protective effect by more effective clearance of amyloid β 1-42 deposits (Ladu et al., *J Biol. Chem.,* **269,** 23403-23406, 1994; Ladu et al., *J. Biol. Chem.,* **270,** 9039-42, 1995).

The role of other plaque components is not as clear, though recent studies (Oda et al., *Exptl. Neurology,* **136**, 22-31, 1995) have shown that apo J (clusterin) can significantly enhance the toxicity of aggregated amyloid β 1-42 *in vitro.* It also has been reported that HSPG enhances the toxicity of amyloid β 1-40 when injected into rat brain (Snow et al., *Soc. Neurosci. Abstr.,* **18,** 1465, 1992). Wright et al. (*Ann Neurol.,* **34,** 373-384, 1993) demonstrated that amyloid plaques from Alzheimer's disease brain contain significant levels of BChE, while amyloid plaques from elderly non-demented individuals do not. The acute phase inflammatory protein ACT also is upregulated in Alzheimer's disease brain, and it is known to associate with the N-terminal 16 residues of amyloid β. Ma et al. (Ma et al., *Nature,* **372**, 92-94, 1994) have reported that ACT can enhance the aggregation of amyloid β 1-42, and these authors speculate that the enhanced aggregation contributes to its neurotoxicity.

Amyloid β Cellular Responses and *In Vivo* Pathology. Beyond the plaques and tangles that are the hallmarks of Alzheimer's disease, it is clear that a range of cellular responses has been induced, both in neurons and in accompanying glial cells. At a biochemical level, hyperphosphorylation of the tau protein is evident, resulting from perturbation of the kinase/phosphatase balance. At a transcriptional level, a variety of genes are activated to produce a spectrum of proteins not normally present or only present at lower levels in the brain. There also is significant evidence that inflammatory processes have been activated. In particular, tau phosphorylation has been documented to be induced by aggregated amyloid β 1-42 in differentiated SH-SY5Y cells (Lambert et al., *J. Neurosci. Res.,* **39**, 377-384, 1994), and this result has been confirmed in a more recent report by Busciglio et al. (*Neuron, **14**,* 879-88, 1995), in which amyloid β activated tau phosphorylation in cultured primary rat hippocampal neurons.

Fibrillar Amyloid β and Neurodegeneration in Alzheimer's Disease. The mechanism by which amyloid β 1-42 causes Alzheimer's disease has not been elucidated, but the literature contains more than.200 studies of amyloid β neurotoxicity, many of which have been reviewed recently (e.g., Yankner et al., *Neuron,* **16**, 921-32, 1996; Iversen et al., *Biochemical Journal,* **311**, 1-16, 1995). The consensus view is that in order for amyloid β to be toxic, it must assemble into fibrillar structures (Pike et al., *J. Neurosci.,* **13,** 1676-87, 1993). Solutions containing only monomeric amyloid β have repeatedly been demonstrated to have no deleterious effect on neurons in culture. Furthermore, studies have correlated the formation of amyloid β-sheet containing fibrils and the timing and extent of toxicity using techniques such as circular dichroism and electron microscopy (Simmons et al., *Molecular Pharmacology,* **45**, 373-9, 1994). One study concluded explicitly that amyloid β must exist in fibrillar form in order for it to be toxic (Lorenzo et al., *Proc. Natl. Acad. Sci. USA,* **91,** 12243-12247, 1994). Despite this consensus regarding amyloid β structure and activity, there continues to be a problem of reproducibility of published experimental work involving amyloid toxicity (Brining, *Neurobiology of Aging,* **18**, 581-589, 1997), and widespread variability of activity obtained with different batches of amyloid, or even the same batch of amyloid handled in slightly different ways, in spite of identical chemical composition (May et al., *Neurobiology of Aging,* **13**, 1676-87, 1993). This has raised questions regarding the precise structures of amyloid β that are responsible for its activity.

The present invention seeks to overcome the problems in the prior art. Accordingly, it is an object of the present invention to provide a new composition of matter, amyloid β peptide assembled into soluble globular non-fibrillar oligomeric structures (ADDLs), that unexpectedly are neurotoxic. These and other objects and advantages of the present invention, as well as additional inventive features, will be apparent from the following description.

### BRIEF DESCRIPTION OF THE FIGURES

**Figure 1** is a computer-generated image of a densitometer-scanned silver-stained polyacrylamide gel which shows the ADDLs electrophoresing with a primary band corresponding to about 30 kD, a less abundant band corresponding to about 17 kD, and no evidence of fibrils or aggregates.
**Figure 2** is a computer-generated image of a densitometer-scanned Coomassie-stained SDS-polyacrylamide gel which shows ADDLs electrophoresing with a primary band (upper doublet) corresponding to a size of about 17 to about 22 kD, and with another band (lower dark band) indicating abundant 4 kD monomer present, presumably a breakdown product. *Lanes:* first, molecular size markers; second ADDL preparation; third, heavier loading of ADDL preparation.
**Figure 3** is a representative computer-generated image of AFM analysis of ADDL-containing "fraction 3" (fractionated on a Superdex 75 gel filtration column).
**Figure 4** is a computer-generated image of a densitometer-scanned Coomassie-stained SDS-polyacrylamide gradient gel of ADDLs prepared by coincubation with clusterin (*lane A*) or cold F12 media (*lane B*), and of ADDLs prepared by coincubation with clusterin and which passed through a Centricon 10 kD cut-off membrane (*lane C*) or were retained by a Centricon 10 kD cut-off membrane (*lane D*): **MW**, molecular size markers.
**Figure 5** is a graph of ADDL concentration measured as amyloid β 1-42 concentration (nM) vs. % dead cells for brain slices from mice treated with the ADDL preparations.
**Figure 6** is a bar chart showing % MTT reduction for control PC 12 cells not exposed to ADDLs ("Cont."), PC 12 cells exposed to clusterin alone ("Apo J"), PC 12 cells exposed to monomeric Aβ ("Aβ"), PC12 cells exposed to amyloid β coaggregated with clusterin and aged one day ("Aβ:Apo J").
**Figure 7** is a FACScan showing fluorescence intensity (0-170) versus events (0-300) for B 103 cells not exposed to ADDLs (unshaded peak) and B103 cells bound to fluorescent labeled ADDLs (shaded peak).
**Figure 8** is a FACScan showing fluorescence intensity (0-200) versus events (0-300) for hippocampal cells not exposed to ADDLs (unshaded peak, "-ADDLs") and hippocampal cells bound to fluorescent labeled ADDLs (shaded peak, "+ADDLs").
**Figure 9** is a bar chart of percent maximum ADDL binding or ADDL-evoked death for B103 cells that either have been not exposed ("-") or coexposed ("+") to the peptides released by trysinization of B103 cells.
**Figure 10** is a graph of relative ADDL concentration vs. % dead cells for brain slices from mice treated with the ADDL preparations. To determine relative concentration, an initial concentration of 10 µM Aβ protein was employed to form ADDLs at the highest data point (point "16"), this was subsequently diluted to ½ (point "8"), ¼ (point "4"), and the like.
**Figure 11** is a bar chart showing optical density obtained in the ADDL binding ELISA assay wherein B103 cells were coincubated with ADDLs and 6E10 antibody ("cells, ADDL, 6E10" bar), B103 cells were coincubated with ADDLs and ("cells, ADDL" bar), B103 cells were coincubated with 6E10 antibody ("cells, 6E10" bar), B103 cells were incubated alone ("cells" bar), 6E10 antibody was incubated alone ("6E10" bar), or the optical density of diluent was read ("blank" bar).
**Figure 12** is a bar chart of % dead cells in either *fyn* +/+ (wild type, "Fyn +"; crosshatched bars) or *fyn* -/- (knockout, "Fyn -"; solid bars) mice either not treated ("Medium") or contacted with ADDLs ("ADDLs").
**Figure 13** is a graph of Aβ concentration (µM) versus activated glia (number) obtained upon incubation of astrocytes with ADDLs (filled triangles) or Aβ 17-42 (filled squares).
**Figure 14** is a graph of time (minutes) versus % baseline cell body spike amplitude for control mice not treated with ADDLs (filled triangles) or mice treated with ADDLs (filled squares).
**Figure 15** is a graph of time (minutes) versus mean spike amplitude for control rat hippocampal slices not exposed to ADDLs (filled triangles) versus rat hippocampal slices exposed to ADDLs (filled squares).

### SUMMARY OF THE INVENTION

The invention encompasses a new composition of matter, termed amyloid beta-derived dementing ligands or amyloid beta-derived diffusible ligands (ADDLs). ADDLs consist of amyloid β peptide assembled into soluble non-fibrillar oligomeric structures that are capable of activating specific cellular processes. Another aspect of the invention consists of methods for assaying the formation, presence, receptor protein binding and cellular activities of ADDLs. The invention further encompasses assay methods and methods of identifying compounds that modulate (e.g., increase or decrease) the formation and/or activity of ADDLs. Such compounds can be employed in the treatment of diseases, disorders, or conditions due to the effects of the ADDLs.

### DETAILED DESCRIPTION OF THE INVENTION

It has been discovered that in neurotoxic samples of amyloid β not only do fibrillar structures exist, but also, unexpectedly, some small globular protein structures exist that appear to be responsible for the neurotoxicity. Using novel methods, samples that contain predominantly these soluble globular protein assemblies and no fibrillar structures have been generated as described herein. In heterogeneous samples prepared by various methods, the removal of the larger, fibrillar forms of amyloid β by centrifugation does not remove these soluble globular assemblies of amyloid β in the supernatant fractions. These supernatant fractions exhibit significantly higher neurotoxicity than unfractionated amyloid β samples aggregated under literature conditions. These novel and unexpected neurotoxic soluble globular forms are referred to herein as amyloid β-derived dementing ligands or amyloid β-derived diffusible ligands (ADDLs). Samples of amyloid β that had been "aged" under standard literature conditions (e.g., Pike et al., *J. Neurosci.,* **13,** 1676-1687, 1993) for more than three weeks lose their neurotoxicity, even though these samples contain predominantly fibrillar structures with few or no ADDLs. This discovery that the globular ADDLs are neurotoxic is particularly surprising since current thinking holds that it is fibril structures that constitute the toxic form of amyloid β (Lorenzo et al., *Proc. Natl. Acad. Sci. USA,* **91**, 12243-12247, 1994; Howlett et al., *Neurodegen,* **4**, 23-32, 1995).

The ADDLs can be formed in *vitro.* When a solution (e.g., a DMSO solution) containing monomeric amyloid β 1-42 (or other appropriate amyloid β, as further described herein) is diluted into cold tissue culture media (e.g., F12 cell culture media), then allowed to incubate at about 4°C for from about 2 to about 48 hours and centrifuged for about 10 minutes at about 14,000g at a temperature of 4°C, the supernatant fraction contains small, soluble oligomeric globules that are highly neurotoxic, e.g., in neuronal cell and brain slice cultures, The ADDLs also can be formed by coincubation of amyloid β with certain appropriate agents, e g., clusterin (a senile plaque protein that also is known as ApoJ).

Atomic force microscope analysis (AFM) of such a supernatant fraction reveals a number of different size globules present in the fraction. These globules fall within the range of from about 47 nm to about 6 2 nm. There may be distinct species of globules falling within this range. Namely, slight variation in height surface results from how the particular species are seated on the mica surface at the time of analysis. Despite this slight variation however, there appear to be two predominant sizes of globules, i.e., from about 4.9 nm to about 5.4 nm and from about 5.7 nm to about 6 2 nm, that constitute about 50% of the oligomeric structures in a typical sample. There also may be a distinct size species of globule having dimensions of from about 5-3 nm to about 5.7 nm. It appears that the globules of dimensions of from about 4 7 nm to about 6.2 nm on AFM comprise the pentamer and hexamer form of oligomeric amyloid β protein. The AFM size globules of from about 4.2 nm to about 4-7 nm appear to correspond to the Aβ tetramer. The size globules of from about 3.4 nm to about 4.0 nm to appear to correspond to trimcr. The size globules of from about 2.8 nm to about 3.4 nm correspond to dimer (Roher et al., *J*. *Biol. Chem.,* **271**, 20631-20635, 1996). The Aβ monomer AFM size ranges from about 0.8 nm to about 1.8 - 2.0 nm. Monomeric and dimeric amyloid β are not neurotoxic in neuronal cell cultures or in the organotypic brain slice cultures.

Thus, the present invention provides an isolated and homogenous, soluble, globular, non-fibrillar amyloid β protein assembly (i.e., ADDL) that comprises 4 to 12 amyloid β 1-42 proteins, wherein the structure exhibits neurotoxicity and does not contain amyloid β 1-40 protein. In particular, the invention provides an isolated amyloid β protein assembly wherein the assembly preferably comprises an oligomeric form selected from the group consisting of tetramer, pentamer, and hexamer The protein assembly optimally exhibits neurotoxic activity. Amyloid β 1-42 with biocytin at position 1 can be employed. Amyloid β 1-42 with a cysteine at the N-terminus also can be employed.

Desirably, the isolated amyloid β protein assembly according to the invention comprises globules of dimensions of from 4.7 nm to 6.2 nm as measured by atomic force microscopy, Also, preferably the isolated amyloid β protein assembly comprises globules of dimensions of from 4.9 nm to 54 nm, or from 5.7 nm to 6.2 nm, as measured by atomic force microscopy. In particular, preferably the isolated amyloid β protein assembly according to the invention is such that wherein from 30% to 85%, even more preferably from 40% to 75% of the assembly comprises two predominant sizes of globules, namely, of dimensions of from 4.9 nm to 5.4 nm, and from 5.7 nm to 6.2 nm, as measured by atomic force microscopy. However, it also is desirable that the protein assembly comprises AFM size globules of 5-3 to 5.7 nm.

By non-denaturing gel electrophoresis, the bands corresponding to the ADDLs run at from 26 kD to 28 kD. Under denaturing conditions (e.g., on a 15% SDS-polyacrylamide gel), the ADDLs comprise a band that runs at from 22 kD to 24 kD, and may further comprise a band that runs at 18 to 19 kD. Accordingly, the invention preferably provides an isolated amyloid β protein assembly (i.e., ADDL) that has a molecular weight of from 26 kD to 28 kD as determined by non-denaturing gel electrophoresis. The invention also preferably provides an isolated amyloid β protein assembly (i.e., ADDL) that runs as a band corresponding to a molecular weight of from 22 kD to 24 kD, or 18 to 19 kD, as determined by electrophoresis on a 15% SDS-polyacrylamide gel.

There is also disclosed a method for preparing the isolated soluble non-fibrillar amyloid β protein assembly. This method optionally comprises the steps of:
(a) obtaining a solution of monomeric amyloid β protein;
(b) diluting the protein solution into an appropriate media;
(c) incubating the media resulting from step (b) at about 4°C;
(d) centrifuging the media at about 14,000 g at about 4°C; and
(e) recovering the supernatant resulting from the centrifugation as containing the amyloid β protein assembly. In step (c) of this method, the solution desirably is incubated for from about 2 hours to about 48 hours, especially from about 12 hours to about 48 hours, and most preferably from about 24 hours to about 48 hours. In step (d) of this method, the centrifugation preferably is carried out for from about 5 minutes to about 1 hour, especially for from about 5 minutes to about 30 minutes, and optimally for about 10 minutes. Generally, however, this is just a precautionary measure to remove any nascent fibrillar or protofibrillar structures and may not be necessary, particularly where long-term stability of the ADDL preparation is not an issue.

The Aβ protein is diluted in step (b) desirably to a final concentration ranging from about 5 nM to about 500 µM, particularly from about 5µM to about 300 µM, especially at about 100 µM. The "appropriate media" into which the Aβ protein solution is diluted in step (b) preferably is any media that will support, if not facilitate, ADDL formation. In particular, F12 media (which is commercially available as well as easily formulated in the laboratory) is preferred for use in this method of the invention. Similarly, "substitute F12 media" also desirably can be employed. Substitute F12 media differs from F12 media that is commercially available or which is formulated in the laboratory. According to the invention, substitute F12 media preferably comprises the following components: N, N-dimethylglycine, D-glucose, calcium chloride, copper sulfate pentahydrate, iron(II) sulfate heptahydrate, potassium chloride, magnesium chloride, sodium chloride, sodium bicarbonate, disodium hydrogen phosphate, and zinc sulfate heptahydrate.

In particular, synthetic F12 media according to the invention optionally comprises: N, N-dimethylglycine (from about 600 to about 850 mg/L), D-glucose (from about 1.0 to about 3.0 g/L), calcium chloride (from about 20 to about 40 mg/L), copper sulfate pentahydrate (from about 15 to about 40 mg/L), iron(II) sulfate heptahydrate (from about 0.4 to about 1.2 mg/L), potassium chloride (from about 160 to about 280 mg/L), magnesium chloride (from about 40 to about 75 mg/L), sodium chloride (from about 6.0 to about 9.0 g/L), sodium bicarbonate (from about 0.75 to about 1.4 g/L), disodium hydrogen phosphate (from about 120 to about 160 mg/L), and zinc sulfate heptahydrate (from about 0.7 to about 1.1 mg/L). Optimally, synthetic F12 media according to the invention comprises: N, N-dimethylglycine (about 766 mg/L), D-glucose (about 1.802 g/L), calcium chloride (about 33 mg/L), copper sulfate pentahydrate (about 25 mg/L), iron(II) sulfate heptahydrate (about 0.8 mg/L), potassium chloride (about 223 mg/L), magnesium chloride (about 57 mg/L), sodium chloride (about 7.6 g/L), sodium bicarbonate (about 1.18 g/L), disodium hydrogen phosphate (about 142 mg/L), and zinc sulfate heptahydrate (about 0.9 mg/L). Further, the pH of the substitute F12 media preferably is adjusted, for instance, using 0.1 M sodium hydroxide, desirably to a pH of from about 7.0 to about 8.5, and preferably a pH of about 8.0.

The foregoing method further desirably can be carried out by forming the slowly-sedimenting protein assembly in the presence of an appropriate agent, such as clusterin. This is done, for instance, by adding clusterin in step (c), and, as set out in the Examples which follow.

If the ADDLs are prepared by the incorporation of 10% biotinylated amyloid β 1-42 (or other appropriate biotinylated amyloid β protein), they can be utilized in a receptor binding assay using neural cells and carried out, for instance, on a fluorescence activated cell sorting (FACS) instrument, with labeling by a fluorescent avidin conjugate. Alternately, instead of incorporating biotin in the amyloid β protein, another reagent capable of binding the ADDL to form a fluorescently labeled molecule, and which may already be part of a fluorescent-labeled conjugate, can be employed. For instance, the protein assembly can be formed such that the amyloid protein includes another binding moiety, with "binding moiety" as used herein encompassing a molecule (such as avidin, streptavidin, polylysine, and the like) that can be employed for binding to a reagent to form a fluorescently-labeled compound or conjugate. The "fluorescent reagent" to which the protein assembly binds need not itself fluoresce directly, but instead may merely be capable of fluorescence through binding to another agent. For example, the fluorescent reagent which binds the protein assembly can comprise a β amyloid specific antibody (e.g., 6E10), with fluorescence generated by use of a fluorescent secondary antibody.

Along with other experiments, FACSscan analysis of the rat CNS B103 cells was done without and with ADDL incubation. Results of these and further studies confirm that binding to the cell surface is saturable, and brief treatment with trypsin selectively removes a subset of cell surface proteins and eliminates binding of ADDLs. Proteins that are cleavable by brief treatment with trypsin from the surface of B103 cells also prevent ADDL binding to B103 cells or cultured primary rat hippocampal neurons. These results all support that the ADDLs act through a particular cell surface receptor, and that early events mediated by the ADDLs (i.e., events prior to cell killing) can be advantageously controlled (e.g., for treatment or research) by compounds that block formation and activity (e.g., including receptor binding) of the ADDLs.

Thus, the invention provides a method for identifying compounds that modulate (i.e., either facilitate or block) receptor binding of the ADDL. This method preferably comprises:
(a) contacting separate cultures of neuronal cells with the protein assembly either in the presence or absence of contacting with the test compound;
(b) adding a reagent that binds to the protein assembly, the reagent being fluorescent;
(c) analyzing the separate cell cultures by fluorescence-activated cell sorting; and
(d) comparing the fluorescence of the cultures, with compounds that block receptor binding of the protein assembly being identified as resulting in a reduced fluorescence of the culture, and compounds that facilitate receptor binding being identified as resulting in an increased fluorescence of the culture, as compared to the corresponding culture contacted with the protein assembly in the absence of the test compound. Alternately, instead of adding a fluorescent reagent that in and of itself is able to bind the protein complex, the method desirably is carried out wherein the protein assembly is formed from amyloid β 1-42 protein (or another amyloid β) prepared such that it comprises a binding moiety capable of binding the fluorescent reagent.

Similarly, the method can be employed for identifying compounds that modulate (i.e., either facilitate or block) formation or receptor binding of the protein assembly comprising:
(a) preparing separate samples of amyloid β that either have or have not been mixed with the test compound;
(b) forming the protein assembly in the separate samples;
(c) contacting separate cultures of neuronal cells with the separate samples;
(d) adding a reagent that binds to the protein assembly, the reagent being fluorescent;
(e) analyzing the separate cell cultures by fluorescence-activated cell sorting; and
(f) comparing the fluorescence of the cultures, with compounds that block formation or receptor binding of the protein assembly being identified as resulting in a reduced fluorescence of the culture, and compounds that facilitate formation or receptor binding of the protein assembly being identified as resulting in an increased fluorescence of the culture, as compared to the corresponding culture contacted with the protein assembly in the absence of the test compound. Further, instead of adding a fluorescent reagent that in and of itself is able to bind the protein complex, the method can be carried out wherein the protein assembly is formed from amyloid β protein prepared such that it comprises a binding moiety capable of binding the fluorescent reagent.

The fluorescence of the cultures further optionally is compared with the fluorescence of cultures that have been treated in the same fashion except that instead of adding or not adding test compound prior to formation of the protein assembly, the test compound either is or is not added after formation of the protein assembly. In this situation, compounds that block formation of the protein assembly are identified as resulting in a reduced fluorescence of the culture, and compounds that facilitate formation of the protein assembly are identified as resulting in an increased fluorescence of the culture, as compared to the corresponding culture contacted with the protein assembly in the absence of the test compound, *only* when the compound is added prior to protein assembly.

By contrast, compounds that block receptor binding of the protein assembly are identified as resulting in a reduced fluorescence of the culture, and compounds that facilitate receptor binding of the protein assembly are identified as resulting in an increased fluorescence of the culture, as compared to the corresponding culture contacted with the protein assembly in the absence of the test compound, when the compound is added *either prior to or after* protein assembly.

In a similar fashion, a cell-based assay, particularly a cell-based enzyme-linked immunosorbent assay (ELISA) can be employed in accordance with the invention to assess ADDL binding activity. In particular, the method can be employed to detect binding of the protein assembly to a cell surface receptor. This method preferably comprises:
(a) forming a protein assembly from amyloid β protein;
(b) contacting a culture of neuronal cells with the protein assembly;
(c) adding an antibody (e.g., 6E10) that binds said protein assembly, said antibody including a conjugating moiety (e.g., biotin, or other appropriate agent);
(d) washing away unbound antibody;
(e) linking an enzyme (e.g., horseradish peroxidase) to said antibody bound to said protein assembly by means of said conjugating moiety;
(f) adding a colorless substrate (e.g., ABTS) that is cleaved by said enzyme to yield a color change; and
(g) determining said color change (e.g., spectrophotometrically) or the rate of the color change as a measure of receptor binding of said protein assembly. As earlier described, the antibody can be any antibody capable of detecting ADDLs (e.g., an antibody directed to an exposed site on amyloid β), and the antibody conjugating moiety can be any agent capable of linking a means of detection (e.g., an enzyme). The enzyme can be any moiety (e.g., perhaps even other than a protein) that provides a means of detecting (e.g., color change due to cleavage of a substrate), and further, can be bound (e.g., covalent or noncovalent) to the antibody bound to the protein assembly by means of another moeity (e.g., a secondary antibody). Also, preferably according to the invention the cells are adhered to a solid substrate (e.g., tissue culture plastic) prior to the conduct of the assay. It goes without saying that desirably step (b) should be carried out as described herein such that ADDLs are able to bind to cells. Similarly, preferably step (c) should be carried out for a sufficient length of time (e.g., from about 10 minutes to about 2 hours, desirably for about 30 minutes) and under appropriate conditions (e.g., at about room temperature, preferably with gentle agitation) to allow antibody to bind to ADDLs. Further, appropriate blocking steps can be carried out such as are known to those skilled in the art using appropriate blocking reagents to reduce any nonspecific binding of the antibody. The artisan is familiar with ELISAs and can employ modifications to the assay such as are known in the art.

The assay desirably also can be carried out so as to identify compounds that modulate (i.e., either facilitate or block) formation or receptor binding of the protein assembly. In this method, as in the prior-described assays for test compounds, the test compound is either added to the ADDL preparation, prior to the contacting of the cells with the ADDLs. This assay thus can be employed to detect compounds that modulate formation of the protein assembly (e.g., as previously described). Moreover, the test compound can be added to the ADDL preparation prior to contacting the cells (but after ADDL formation), or to the cells prior to contact with ADDLs. This method (e.g., as previously described) can be employed to detect compounds that modulate ADDL binding to the cell surface. Also, a test compound can be added to the mixture of cells plus ADDLs. This method (e.g., as previously described) can be employed to detect compounds that impact on ADDL-mediated events occurring downstream of ADDL receptor binding. The specificity of the compounds for acting on an ADDL-mediated downstream effect can be confirmed, for instance, by simply adding the test compound in the absence of any coincubation with ADDLs. Of course, further appropriate controls (e.g., as set forth in the following Examples and as known to those skilled in the art) should be included with all assays.

This information on compounds the modulate (i.e., facilitate or block) formation and/or activity including receptor binding of the protein assembly can be employed in the research and treatment of ADDL-mediated diseases, conditions, or disorders. The methods of the invention can be employed to investigate the activity and neurotoxicity of the ADDLs themselves. For instance, when 20 nL of the ADDL preparation was injected into the hippocampal region of an adult mouse 60-70 minutes prior to the conduct of a long-term potentiation (LTP) experiment (e.g. Namgung et al., *Brain Research,* **689**, 85-92, 1995), the stimulation phase of the experiment occurred in a manner identical with saline control injections, but the consolidation phase showed a significant, continuing decline in synaptic activity as measured by cell body spike amplitude, over the subsequent 2 hours, compared with control animals, in which synaptic activity remained at a level comparable to that exhibited during the stimulation phase. Analysis of brain slices after the experiment indicated that no cell death had occurred. These results, as well as other described in the following Examples, confirm that ADDL treatment compromised the LIP response. This indicates that ADDLs contribute to the compromised learning and memory observed in Alzheimer's disease by interference with neuronal signaling processes, rather than by the induction of nerve cell death.

Additional information on the effects of ADDLs (either in the presence or absence of test compounds that potentially modulate ADDL formation and/or activity) can be obtained using the further assays according to the invention. For instance, there is also disclosed a method for assaying the effects of ADDLs that preferably comprises:
(a) administering the protein assembly to the hippocampus of an animal;
(b) applying an electrical stimulus; and
(c) measuring the cell body spike amplitude over time to determine the long-term potentiation response. The method optionally is carried out wherein the long-term potentiation response of the animal is compared to the long-term potentiation response of another animal treated in the same fashion except having saline administered instead of protein assembly prior to application of the electrical stimulus. This method further can be employed to identify compounds that modulate (i.e., increase or decrease) the effects of the ADDLs, for instance, by comparing the LTP response in animals administered ADDLs either alone, or, in conjunction with test compounds.

Along these lines, there is also disclosed a method for identifying compounds that modulate the effects of the ADDL protein assembly. The method preferably comprises:
(a) administering either saline or a test compound to the hippocampus of an animal;
(b) applying an electrical stimulus;
(c) measuring the cell body spike amplitude over time to determine the long-term potentiation response; and
(d) comparing the long-term potentiation response of animals having saline administered to the long-term potentiation response of animals having test compound administered. The method further optionally comprises administering protein assembly to the hippocampus either before, along with, or after administering the saline or test compound.

Similarly, there is also disclosed a method for identifying compounds that modulate (i.e., either increase or decrease) the neurotoxicity of the ADDL protein assembly, which method comprises:
(a) contacting separate cultures of neuronal cells with the protein assembly either in the presence or absence of contacting with the test compound;
(b) measuring the proportion of viable cells in each culture: and
(c) comparing the proportion of viable cells in each culture. Compounds that block the neurotoxicity of the protein assembly are identified, for example, as resulting in an increased proportion of viable cells in the culture as compared to the corresponding culture contacted with the protein assembly in the absence of the test compound. Compounds that increase the neurotoxicity of the protein assembly are identified, for example, as resulting in a reduced portion of viable cells in the culture as compared to the corresponding culture contacted with the protein assembly in the presence of the test compound.

The methods of the invention also can be employed in detecting in test materials the ADDLs (e.g., as part of research, diagnosis, and/or therapy). For instance, ADDLs bring about a rapid morphological change in serum-starved B103 cells, and they also activate Fyn kinase activity in these cells within 30 minutes of ADDL treatment (data not shown). ADDLs also induce rapid complex formation between Fyn and focal adhesion kinase (FAK; Zhang et al, *Neurosci. Letters,* **211**, 1-4, 1996), and translocating of several phosphorylated proteins and Fyn-Fak complex to a Triton-insoluble fraction (Berg et al., *J*. *Neurosci. Res.,* **50,** 979-989, 1997) This suggests that Fyn and other activated signaling pathways are involved in the neurodegenerative process induced by ADDLs. This has been continued by experiments in brain slice cultures from genetically altered mice that lack a functional *fyn* gene, where addition of ADDLs resulted in no increased neurotoxicity compared to vehicle controls.

Therefore, compounds that block one or more of Fyn's function, or Fyn relocalization, namely by impacting on ADDLs, may be important neuroprotective drugs for Alzheimer's disease. Similarly, when ADDLs are added to cultures of primary astrocytes, the astrocytes become activated and the mRNA for several proteins, including IL-1, inducible nitric oxide synthase, Apo E, Apo J and α1-antichymotrypsin become elevated. These phenomena desirably are employed in a method for detecting in a test material the ADDL protein assembly. Such methods optionally comprise:
(a) contacting the test material with an antibody (e.g., the 6E10 antibody or another antibody); and
(b) detecting binding to the protein assembly of the antibody.

Similarly, the method desirably can be employed wherein
(a) the test material is contacted with serum-starved neuroblastoma cells (e.g., B103 neuroblastoma cells); and
(b) morphological changes in the cells are measured by comparing the morphology of the cells against neuroblastoma cells that have not been contacted with the test material.

The method also preferably can be employed wherein:
(a) the test material is contacted with brain slice cultures; and
(b) brain cell death is measured as compared against brain slice cultures that have not been contacted with the test material. The method further desirably can be conducted wherein:

(a) the test material is contacted with neuroblastoma cells (e.g., B103 neuroblastoma cells); and
(b) increases in *fyn* kinase activity are measured by comparing *fyn* kinase activity in the cells against *fyn* kinase activity in neuroblastoma cells that have not been contacted with said test material. In particular, Fyn kinase activity can be compared making use of a commercially available kit (e.g., Kit #QIA-28 from Oncogene Research Products, Cambridge, MA) or using an assay analogous to that described in Borowski et al., *J. Biochem.* (Tokyo), **115**, 825-829, 1994.

In yet another preferred embodiment of the method of detecting ADDLs in test material, the method desirably comprises:
(a) contacting the test material with cultures of primary astrocytes; and
(b) determining activation of the astrocytes as compared to cultures of primary astrocytes that have not been contacted with the test material. In a variation of this method, the method optionally comprises:
   (a) contacting the test material with cultures of primary astrocytes; and
   (b) measuring in the astrocytes increases in the mRNA for proteins selected from the group consisting of interleukin-1, inducible nitric oxide synthase, Apo E, Apo J, and α1-antichymotrypsin by comparing the mRNA levels in the astrocytes against the corresponding mRNA levels in cultures of primary astrocytes that have not been contacted with the test material. There are, of course, other methods of assay, and further variations of those described above that would be apparent to one skilled in the art, particularly in view of the specification disclosure herein.

Thus, clearly, the ADDLs according to the present invention have utility *in vitro.* Such ADDLs can be used *inter alia* as a research tool in the study of ADDL binding and interaction within cells and in a method of assaying ADDL activity. Similarly, ADDLs, and studies of ADDL formation, activity and modulation can be employed *in vivo.*

In particular, the compounds identified using the methods of the present invention can be used to treat any one of a number of diseases, disorders, or conditions that result in deficits in cognition or learning (i.e., due to a failure of memory), and/or deficits in memory itself. Such treatment or prevention can be effected by administering compounds that prevent formation and/or activity of the ADDLs, or that modulate (i.e., increase or decrease the activity of, desirably as a consequence of impacting ADDLs) the cell agents with which the ADDLs interact (e.g., so-called "downstream" events). Such compounds having ability to impact ADDLs are referred to herein as "ADDL-modulating compounds". ADDL-modulating compounds not only can act in a negative fashion, but also, in some cases preferably are employed to increase the formation and/or activity of the ADDLs.

Desirably, when employed *in vivo,* the method can be employed for protecting an animal against decreases in cognition, learning or memory due to the effects of the ADDL protein assembly. This method comprises administering a compound that blocks the formation or activity of the ADDLs. Similarly, to the extent that deficits in cognition, learning and/or memory accrue due to ADDL formation and/or activity, such deficits can be reversed or restored once the activity (and/or formation) of ADDLs is blocked. The invention thus preferably provides a method for reversing (or restoring) in an animal decreases in cognition, learning or memory due to the effects of a protein assembly according to the invention. This method preferably comprises blocking the formation or activity of the ADDLs.

In particular, this method desirably can be applied in the treatment or prevention of a disease, disorder, or condition that manifests as a deficit in cognition, learning and/or memory and which is due to ADDL formation or activity, especially a disease, disorder, or condition selected from the group consisting of Alzheimer's disease, adult Down's syndrome (i.e., over the age of 40 years), and senile dementia.

Also, this method desirably can be applied in the treatment or prevention of early deleterious effects on cellular activity, cognition, learning, and memory that may be apparent prior to the development of the disease, disorder, or condition itself, and which deleterious effects may contribute to the development of, or ultimately constitute the disease, disorder, or condition itself. In particular, the method preferably can be applied in the treatment or prevention of the early malfunction of nerve cells or other brain cells that can result as a consequence of ADDL formation or activity. Similarly, the method preferably can be applied in the treatment or prevention of focal memory deficits (FMD) such as have been described in the literature (e.g., Linn et al., *Arch. Neurol.,* **52**, 485-490, 1995), in the event such FMD are due to ADDL formation or activity. The method further desirably can be employed in the treatment or prevention of ADDL-induced aberrant neuronal signaling, impairment of higher order writing skills (e.g., Snowdon et al., *JAMA,* **275**, 528-532, 1996) or other higher order cognitive function, decreases in (or absence of) long-term potentiation, that follows as a consequence of ADDL formation or activity.

According to this invention, "ADDL-induced aberrant neuronal signaling" can be measured by a variety of means. For instance, for normal neuronal signaling (as well as observation of a long-term potentiation response), it appears that among other things, Fyn kinase must be activated, Fyn kinase must phosphorylate the NMDA channel (Miyakawa et al., *Science.* **278,** 698-701, 1997; Grant, *JPhysiol Paris,* **90*,*** 337-338, 1996), and Fyn must be present in the appropriate cellular location (which can be impeded by Fyn-FAK complex formation, for instance, as occurs in certain cytoskeletal reorganizations induced by ADDL). Based on this, ADDL-induced aberrant neuronal signaling (which is a signaling malfunction that is induced by aberrant activation of cellular pathways by ADDLs) and knowledge thereof can be employed in the methods of the invention, such as would be obvious to one skilled in the art. For instance, ADDL-induced aberrant cell signaling can be assessed (e.g., as a consequence of contacting nerve cells with ADDLs, which may further be conducted in the presence or absence of compounds being tested for ADDL-modulating activity) using any of these measures, or such as would be apparent to one skilled in the art, e.g., Fyn kinase activation (or alteration thereof), Fyn-FAK complex formation (or alteration thereof), cytoskeletal reorganization (or alteration thereof), Fyn kinase subcellular localization (or alteration thereof), Fyn kinase phosphorylation of the NMDA channel (or alteration thereof).

Also, the ADDLs themselves may be applied in treatment. It has been discovered that these novel assemblies described herein have numerous unexpected effects on cells that conceivably can be applied for therapy. For instance, ADDLs activate endothelial cells, which endothelial cells are known, among other things to interact with vascular cells. Along these lines, ADDLs could be employed, for instance, in wound healing. Also, by way of example, Botulinum Toxin Type A (BoTox) is a neuromuscular junction blocking agent produced by the bacterium *Clostridium botulinum* that acts by blocking the release of the neurotransmitter acetylcholine. Botox has proven beneficial in the treatment of disabling muscle spasms, including dystonia. ADDLs themselves theoretically could be applied to either command neural cell function or, to selectively destroy targeted neural cells (e.g., in cases of cancer, for instance of the central nervous system, particularly brain). ADDLs appear further advantageous in this regard given that they have very early effects on cells, and given that their effect on cells (apart from their cell killing effect) appears to be reversible.

As discussed above, the ADDL-modulating compounds of the present invention, as well as ADDLs themselves, can be employed to contact cells either *in vitro* or *in vivo.* According to the invention, a cell can be any cell, and, preferably, is a eukaryotic cell. A eukaryotic cell is a cell typically that possesses at some stage of its life a nucleus surrounded by a nuclear membrane. Preferably the eukaryotic cell is of a multicellular species (e.g., as opposed to a unicellular yeast cell), and, even more preferably, is a mammalian (optionally human) cell. However, the method also can be effectively carried out using a wide variety of different cell types such as avian cells, and mammalian cells including but not limited to rodent, primate (such as chimpanzee, monkey, ape, gorilla, orangutan, or gibbon), feline, canine, ungulate (such as ruminant or swine), as well as, in particular, human cells. Preferred cell types are cells formed in the brain, including neural cells and glial cells. An especially preferred cell type according to the invention is a neural cell (either normal or aberrant, e.g., transformed or cancerous). When employed in tissue culture, desirably the neural cell is a neuroblastoma cell.

A cell can be present as a single entity, or can be part of a larger collection of cells. Such a "larger collection of cells" can comprise, for instance, a cell culture (either mixed or pure), a tissue (e.g., neural or other tissue), an organ (e.g., brain or other organs), an organ system (e.g., nervous system or other organ system), or an organism (e.g., mammal, or the like). Preferably, the organs/tissues/cells of interest in the context of the invention are of the central nervous system (e.g., are neural cells).

Also, according to the invention "contacting" comprises any means by which these agents physically touch a cell. The method is not dependent on any particular means of introduction and is not to be so construed. Means of introduction are well known to those skilled in the art, and also are exemplified herein. Accordingly, introduction can be effected, for instance, either *in vitro* (e.g., in an *ex vivo* type method of therapy or in tissue culture studies) or *in vivo.* Other methods also are available and are known to those skilled in the art.

Such "contacting" can be done by any means known to those skilled in the art, and described herein, by which the apparent touching or mutual tangency of the ADDLs and ADDL-modulating compounds and the cell can be effected. For instance, contacting can be done by mixing these elements in a small volume of the same solution. Optionally, the elements further can be covalently joined, e.g., by chemical means known to those skilled in the art, or other means, or preferably can be linked by means of noncovalent interactions (e.g., ionic bonds, hydrogen bonds, Van der Waals forces, and/or nonpolar interactions). In comparison, the cell to be affected and the ADDL or ADDL-modulating compound need not necessarily be brought into contact in a small volume, as, for instance, in cases where the ADDL or ADDL-modulating compound is administered to a host, and the complex travels by the bloodstream or other body fluid such as cerebrospinal fluid to the cell with which it binds. The contacting of the cell with a ADDL or ADDL-modulating compound sometimes is done either before, along with, or after another compound of interest is administered. Desirably this contacting is done such that there is at least some amount of time wherein the coadministered agents concurrently exert their effects on a cell or on the ADDL.

One skilled in the art will appreciate that suitable methods of administering an agent (e.g., an ADDL or ADDL-modulating compound) of the present invention to an animal for purposes of therapy and/or diagnosis, research or study are available, and, although more than one route can be used for administration, a particular route can provide a more immediate and more effective reaction than another route. Pharmaceutically acceptable excipients also are well-known to those who are skilled in the art, and are readily available. The choice of excipient will be determined in part by the particular method used to administer the agent. Accordingly, there is a wide variety of suitable formulations for use in the context of the present invention. The following methods and excipients are merely exemplary and are in no way limiting.

Formulations suitable for oral administration can consist of (a) liquid solutions, such as an effective amount of the compound dissolved in diluents, such as water, saline, or orange juice; (b) capsules, sachets or tablets, each containing a predetermined amount of the active ingredient, as solids or granules; (c) suspensions in an appropriate liquid; and (d) suitable emulsions. Tablet forms can include one or more of lactose, mannitol, corn starch, potato starch, microcrystalline cellulose, acacia, gelatin, colloidal silicon dioxide, croscarmellose sodium, talc, magnesium stearate, stearic acid, and other excipients, colorants, diluents, buffering agents, moistening agents, preservatives, flavoring agents, and pharmacologically compatible excipients. Lozenge forms can comprise the active ingredient in a flavor, usually sucrose and acacia or tragacanth, as well as pastilles comprising the active ingredient in an inert base, such as gelatin and glycerin, emulsions, gels, and the like containing, in addition to the active ingredient, such excipients as are known in the art.

An agent of the present invention, alone or in combination with other suitable ingredients, can be made into aerosol formulations to be administered via inhalation. These aerosol formulations can be placed into pressurized acceptable propellants, such as dichlorodifluoromethane, propane, nitrogen, and the like. They also can be formulated as pharmaceuticals for non-pressured preparations such as in a nebulizer or an atomizer.

Formulations suitable for parenteral administration are preferred according to the invention and include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The formulations can be presented in unit-dose or multi-dose sealed containers, such as ampules and vials, and can be stored in a freeze-dried (lyophilized) condition requiring only the addition of the sterile liquid excipient, for example, water, for injections, immediately prior to use. Extemporaneous injection solutions and suspensions can be prepared from sterile powders, granules, and tablets of the kind previously described.

The dose administered to an animal, particularly a human, in the context of the present invention will vary with the agent of interest, the composition employed, the method of administration, and the particular site and organism being treated. However, preferably a dose corresponding to an effective amount of an agent (e.g., an ADDL or ADDL-modulating compound according to the invention) is employed. An "effective amount" is one that is sufficient to produce the desired effect in a host, which can be monitored using several end-points known to those skilled in the art. Some examples of desired effects include, but are not limited to, an effect on learning, memory, LTP response, neurotoxicity, ADDL formation, ADDL receptor binding, antibody binding, cell morphological changes, Fyn kinase activity, astrocyte activation, and changes in mRNA levels for proteins such as interleukin-1, inducible nitric oxide synthase, ApoE, ApoJ, and α1-antichymotrypsin. These methods described are by no means all-inclusive, and further methods to suit the specific application will be apparent to the ordinary skilled artisan.

Moreover, with particular applications (e.g., either *in vitro* or *in vivo*) the actual dose and schedule of administration of ADDLs or ADDL-modulating compounds can vary depending on whether the composition is administered in combination with other pharmaceutical compositions, or depending on interindividual differences in pharmacokinetics, drug disposition, and metabolism. Similarly, amounts can vary in *in vitro* applications depending on the particular cell type utilized or the means or solution by which the ADDL or ADDL-modulating compound is transferred to culture. One skilled in the art easily can make any necessary adjustments in accordance with the requirements of the particular situation.

### Examples

The foregoing descriptions (as well as those which follow) are exemplary only. Other applications of the method and constituents of the present invention will be apparent to one skilled in the art. Thus, the following examples further illustrate the present invention but, of course, should not be construed as in any way limiting the scope.

### Example 1: Preparation of Amyloid β Oligomers

According to the invention, ADDLs were prepared by dissolving 1 mg of solid amyloid β 1-42 (e.g., synthesized as described in Lambert et al., *J. Neurosci. Res.,* **39**, 377-395, 1994) in 44 µL of anhydrous DMSO. This 5 mM solution then was diluted into cold (4°C) F12 media (Gibco BRL, Life Technologies) to a total volume of 2.20 mL (50-fold dilution), and vortexed for about 30 seconds. The mixture was allowed to incubate at from about 0°C to about 8°C for about 24 hours, followed by centrifugation at 14,000g for about 10 minutes at about 4°C. The supernatant was diluted by factors of 1:10 to 1:10,000 into the particular defined medium, prior to incubation with brain slice cultures, cell cultures or binding protein preparations. In generalh, however, ADDLs were formed at a concentration of Aβ protein of 100 µM. Typically, the highest concentration used for experiments is 10 µM and, in some cases, ADDLs (measured as initial Aβ concentration) were diluted (e.g., in cell culture media) to 1 nM. For analysis by atomic force microscopy (AFM), a 20 µL aliquot of the 1:100 dilution was applied to the surface of a freshly cleaved mica disk and analyzed. Other manipulations were as described as follows, or as is apparent.

Alternately, ADDL formation was carried out as described above, with the exception that the F12 media was replaced by a buffer (i.e., "substitute F12 media") containing the following components: N, N-dimethylglycine (766 mg/L), D-glucose (1.802 g/L), calcium chloride (33 mg/L), copper sulfate pentahydrate (25 mg/L), iron(II) sulfate heptahydrate (0.8 mg/L), potassium chloride (223 mg/L), magnesium chloride (57 mg/L), sodium chloride (7.6 g/L), sodium bicarbonate (1.18 g/L),disodium hydrogen phosphate (142 mg/L), and zinc sulfate heptahydrate (0.9 mg/L). The pH of the buffer was adjusted to 8.0 using 0.1 M sodium hydroxide.

### Example 2: Crosslinking of Amyloid β Oligomers

Glutaraldehyde has been successfully used in a variety of biochemical systems. Glutaraldehyde tends to crosslink proteins that are directly in contact, as opposed to nonspecific reaction with high concentrations of monomeric protein. In this example, glutaraldehyde-commanded crosslinking of amyloid β was investigated.

Oligomer preparation was carried out as described in example 1, with use of substitute F12 media. The supernatant that was obtained following centrifugation (and in some cases, fractionation) was treated with 0.22 mL of a 25% aqueous solution of glutaraldehyde (Aldrich), followed by 0.67 mL of 0.175 M sodium borohydride in 0.1 M NaOH (according to the method of Levine, *Neurobiology of Aging,* 1995). The mixture was stirred at 4°C for 15 minutes and was quenched by addition of 1.67 mL of 20% aqueous sucrose. The mixture was concentrated 5 fold on a SpeedVac and dialyzed to remove components smaller than 1 kD. The material was analyzed by SDS PAGE. Gel filtration chromatography was carried according to the following: Superose 75PC 3.2/3.0 column (Pharmacia) was equilibrated with filtered and degassed 0.15% ammonium hydrogen carbonate buffer (pH=7.8) at a flow rate of 0.02 mL/min over the course of 18 h at room temperature. The flow rate was changed to 0.04 mL/min and 20 mL of solvent was eluted. 50 microliters of reaction solution was loaded on to the column and the flow rate was resumed at 0.04 mL/min. Compound elution was monitored via UV detection at 220 nm, and 0.5-1.0 mL fractions were collected during the course of the chromatography. Fraction No. 3, corresponding to the third peak of UV absorbance was isolated and demonstrated by AFM to contain globules 4.9 +/- 0.8 nm (by width analysis). This fraction was highly neurotoxic when contacted with brain slice neurons, as described in the examples which follow.

### Example 3: Size Characterization of ADDLs

This example sets forth the size characterization of ADDLs formed as in Example 1, and using a variety of methods (e.g., native gel electophoresis, SDS-polyacrylamide gel electrophoresis, AFM, field flow fractionation, and immunorecognition).

AFM was carried out essentially as described previously (e.g., Stine et al., *J*. *Protein Chem.,* **15,** 193-203, 1996). Namely, images were obtained using a Digital Instruments (Santa Barbara, CA) Nanoscope IIIa Multimode Atomic force microscope using a J-scanner with xy range of 150µ. Tapping Mode was employed for all images using etched silicon TESP Nanoprobes (Digital Instruments). AFM data is analyzed using the Nanoscope IIIa software and the IGOR Pro^{™} waveform analysis software. For AFM analysis, 4 µ scans (i.e., assessment of a 4 µm x 4 µm square) were conducted. Typically, dimensions reported were obtained by section analysis, and where width analysis was employed, it is specified. Section and width analysis are in separate analysis modules in the Nanoscope IIIa software. Generally, for ADDL analysis, there is a systematic deviation between the sizes obtained by section analysis and those obtained by width analysis. Namely, for a 4 µ scan, section analysis yields heights that are usually about 0.5 nm taller, thus resulting in a deviation of about 0.5 nm in the values obtained for the sizes of the globules.

Analysis by gel electrophoresis was carried out on 15% polyacrylamide gels and visualized by Coomassie blue staining. ADDLs were resolved on 4-20% tris-glycine gels under non-denaturing conditions (Novex). Electrophoresis was performed at 20 mA for approximately 1.5 hours. Proteins were resolved with SDS-PAGE as described in Zhang et al., *J. Biol. Chem.,* **269**, 25247-25250, 1994. Protein was then visualized using silver stain (e.g. as described in Sherchenko et al., *Anal. Chem.,* **68**, 850-858, 1996). Gel proteins from both native and SDS gels were transferred to nitrocellulose membranes according to Zhang et al. *(J. Biol. Chem.,* **269,** 25247-50, 1994). Immunoblots were performed with biotinylated 6E10 antibody (Senetak, Inc., St. Louis, Missouri) at 1:5000 and visualized using ECL (Amersham). Typically, gels were scanned using a densitometer. This allowed provision of the computer-generated images of the gels (e.g., versus photographs of the gels themselves).

Size characterization of ADDLs by AFM section analysis (e.g., as described in Stine et al., *J. Protein Chem.,* **15**, 193-203, 1996) or width analysis (Nanoscope III software) indicated that the predominant species were globules of about 4.7 nm to about 6.2 nm along the z-axis. Comparison with small globular proteins (Aβ 1-40 monomer, aprotinin, bFGF, carbonic anhydrase) suggested that ADDLs had mass between 17-42 kD. What appear to be distinct species can be recognized. These appear to correspond to globules of dimensions of from about 4.9 nm to about 5.4 nm, from about 5.4 nm to about 5.7 nm, and from about 5.7 nm to about 6.2 nm. The globules of dimensions of about 4.9-5.4 nm and 5.7-6.2 nm appear to comprise about 50% of globules.

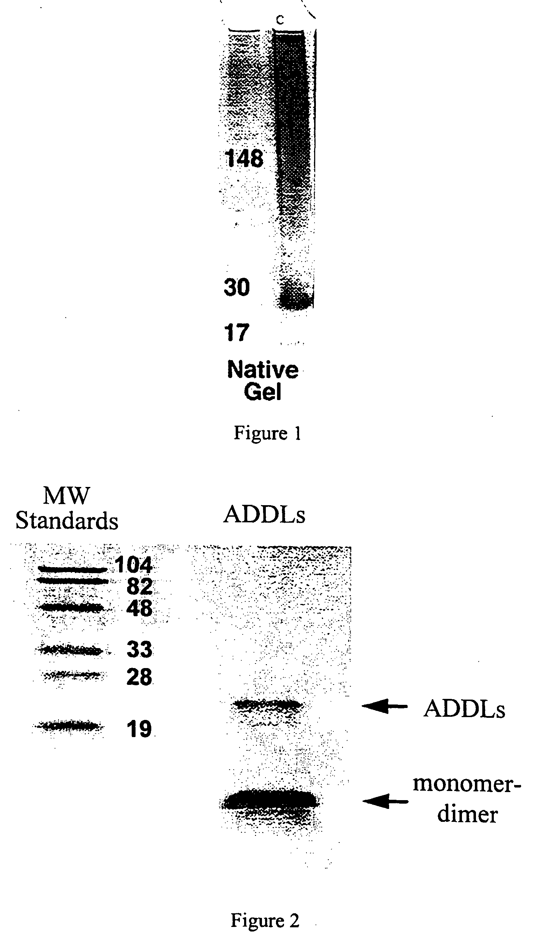

In harmony with the AFM analysis, SDS-PAGE immunoblots of ADDLs identified Aβ oligomers of about 17 kD to about 22 kD, with abundant 4 kD monomer present, presumably a breakdown product. Consistent with this interpretation, non-denaturing polyacrylamide gels of ADDLs show scant monomer, with a primary band near 30 kD, a less abundant band at ~17 kD, and no evidence of fibrils or aggregates. Computer-generated images of a silver stained native gel and a Coomassie stained SDS-polyacrylamide gel are set out in **Figure 1** and **Figure 2,** respectively. The correspondence between the SDS and non-denaturing gels confirms that the small oligomeric size of ADDLs was not due to detergent action. Oligomers seen in ADDL preparations were smaller than clusterin (Mr 80 kD, 40 kD in denatured gels), as expected from use of low clusterin concentrations (1/40 relative to Aβ, which precluded association of Aβ as 1:1 Aβ-clusterin complexes).

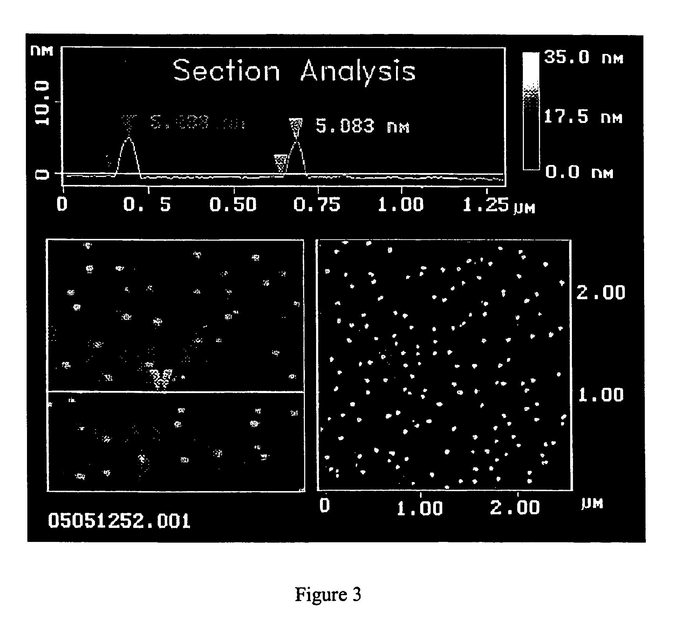

An ADDL preparation according to the invention was fractionated on a Superdex 75 column (Pharmacia, Superose 75PC 3.2/3.0 column). The fraction comprising the ADDLs was the third fraction of UV absorbance eluting from the column and was analyzed by AFM and SDS-polyacryalamide gel electrophoresis. Arepresentative AFM analysis of fraction 3 is depicted in Figure 3. Fractionation resulted in greater homogeneity for the ADDLs, with the majority of the globules having dimensions of from about 4.9 nm to about 5.4 nm. SDS-polyacrylamide gel electrophoresis of the fraction demonstrated a heavy lower band corresponding to the monomer/dimer form of Aβ. As also observed for the non-fractionated preparation of ADDLs, this appears to be a breakdown product of the ADDLs. Heavier loading of the fraction revealed a larger-size broad band (perhaps a doublet). This further confirms the stability of the non-fibrillar oligomeric Aβ structures to SDS.

### Example 4: Clusterin Treatment of Amyloid β

Although it has been proposed that fibrillar structures represent the toxic form of Aβ (Lorenzo et al., *Proc. Natl. Acad. Sci. USA,* **91**, 12243-12247, 1994; Howlett et al., *Neurodegen,* **4**, 23-32, 1995), novel neurotoxins that do not behave as sedimentable fibrils will form when Aβ 1-42 is incubated with low doses of clusterin, which also is known as "Apo J" (Oda et al., *Exper. Neurol.,* **136**, 22-31, 1995; Oda et al., *Biochem. Biophys. Res. Commun.,* **204**, 1131-1136, 1994). To test if these slowly sedimenting toxins might still contain small or nascent fibrils, clusterin-treated Aβ preparations were examined by atomic force microscopy.

Clusterin treatment was carried out as described in Oda et al. (*Exper. Neurol.,* **136**, 22-31, 1995) basically by adding clusterin in the incubation described in Example 1. Alternatively, the starting Aβ 1-42 could be dissolved in 0.1 N HCl, rather than DMSO, and this starting Aβ 1-42 could even have fibrillar structures at the outset. However, incubation with clusterin for 24 hours at room temperature of 37 °C resulted in preparations that were predominantly free of fibrils, consistent with their slow sedimentation. This was confirmed by experiments showing that fibril formation decreases as the amount of clusterin added increases.

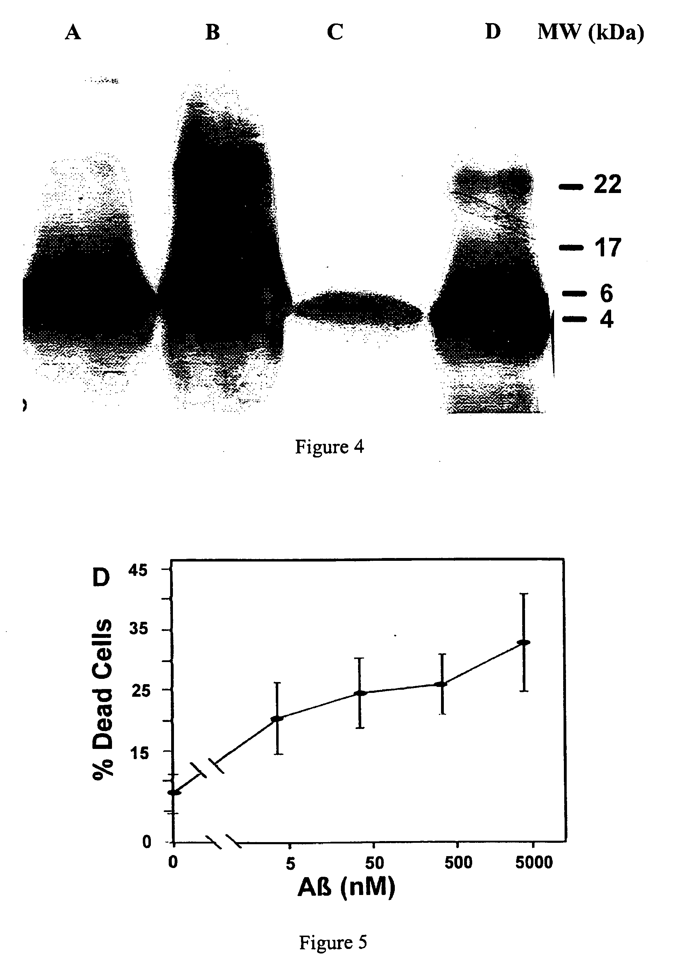

The preparations resulting from clusterin treatment exclusively comprised small globular structures approximately 5-6 nm in size as determined by AFM analysis of ADDLs fractionated on a Superdex 75 gel column. Equivalent results were obtained by conventional electron microscopy. In contrast, Aβ 1-42 that had self-associated under standard conditions (Snyder et al., *Biophys. J.,* **67**, 1216-28, 1994) in the absence of clusterin showed primarily large, non-diffusible fibrillar species. Moreover, the resultant ADDL preparations were passed through a Centricon 10 kD cut-off membrane and analyzed on as SDS-polyacrylamide gradient gel. As can be seen in Figure 4, only the monomer passes through the Centricon 10 filter, whereas ADDLs are retained by the filter. Monomer found after the separation could only be formed from the larger molecular weight species retained by the filter.

These results confirm that toxic ADDL preparations comprise small fibril-free oligomers of Aβ 1-42, and that ADDLs can be obtained by appropriate clusterin treatment of amyloid β.

### Example 5: Physiologic Formation of ADDLs

The toxic moieties in Example 4 could comprise rare structures that contain oligomeric Aβ and clusterin. Whereas Oda et al. *(Exper. Neurol.,* **136**, 22-31, 1995) reported that clusterin was found to increase the toxicity of Aβ 1-42 solutions, others have found that clusterin at stoichiometric levels protects against Aβ 1-40 toxicity (Boggs et al., *J. Neurochem.,* **67**, 1324-1327, 1997). Accordingly, ADDL formation in the absence of clusterin further was characterized in this Example.

When monomeric Aβ 1-42 solutions were maintained at low temperature in an appropriate media, formation of sedimentable Aβ fibrils was almost completely blocked. Aβ, however, did self-associate in these low-temperature solutions, forming ADDLs essentially indistinguishable from those chaperoned by clusterin. Finally, ADDLs also formed when monomeric Aβ solutions were incubated at 37 degrees in brain slice culture medium but at very low concentration (50 nM), indicating a potential to form physiologically. All ADDL preparations were relatively stable and showed no conversion to fibrils during the 24 hour tissue culture experiments.

These results confirm that ADDLs form and are stable under physiological conditions and suggest that they similarly can form and are stable *in vivo.*

### Example 6: ADDLS are Diffusible, Extremely Potent CNS Neurotoxins

Whether ADDLs were induced by clusterin, low temperature, or low Aβ concentration, the stable oligomers that formed were potent neurotoxins. Toxicity was examined in organotypic mouse brain slice cultures, which provided a physiologically relevant model for mature CNS. Brain tissue was supported at the atmosphere-medium interface by a filter in order to maintain high viability in controls.

For these experiments, brain slices were obtained from strains B6 129 F2 and JR 2385 (Jackson Laboratories) and cultured as previously described (Stoppini et al., *J. Neurosci. Meth.,* **37**, 173-182, 1991), with modifications. Namely, an adult mouse was sacrificed by carbon dioxide inhalation, followed by rapid decapitation. The head was emersed in cold, sterile dissection buffer (94 mL Gey's balanced salt solution, pH 7.2, supplemented with 2 mL 0.5M MgCl₂, 2 ml 25% glucose, and 2 mL 1.0 M Hepes), after which the brain was removed and placed on a sterile Sylgard-coated plate. The cerebellum was removed and a mid-line cut was made to separate the cerebral hemispheres. Each hemisphere was sliced separately. The hemisphere was placed with the mid-line cut down and a 30 degree slice from the dorsal side was made to orient the hemisphere. The hemisphere was glued cut side down on the plastic stage of a Campden tissue chopper (previously wiped with ethanol) and emersed in ice cold sterile buffer. Slices of 200 µm thickness were made from a lateral to medial direction, collecting those in which the hippocampus was visible.

Each slice was transferred with the top end of a sterile pipette to a small petri dish containing Dulbecco's Modified Eagle Medium (DMEM) containing 10% fetal calf serum, 2% S/P/F (streptomycin, penicillin, and fungizone; Life Technologies (Gibco, BRL), Gaithersburg, MD), observed with a microscope to verify the presence of the hippocampus, and placed on a Millicell-CM insert (Millipore) in a deep well tissue culture dish (Falcon, 6-well dish). Each well contained 1.0 mL of growth medium, and usually two slices were on each insert. Slices were placed in a incubator (6%CO₂, 100% humidity) overnight. Growth medium was removed and wells were washed with 1.0 mL warm Hanks BSS (Gibco, BRL, Life Technologies). Defined medium (DMEM, N2 supplements, SPF, e.g., as described in Bottenstein et al., *Proc. Natl. Acad. Sci.,* **76**, 514-517, 1979) containing the amyloid β oligomers, with or without inhibitor compounds, was added to each well and the incubation was continued for 24 hours.

Cell death was measured using the **LIVE/DEAD®** assay kit (Molecular Probes, Eugene, OR). This a dual-label fluorescence assay in which live cells are detected by the presence of an esterase that cleaves calcein-AM to calcein, resulting in a green fluorescence. Dead cells take up ethidium homodimer, which intercalates with DNA and has a red fluorescence. The assay was carried out according to the manufacturer's directions at 2 µM ethidium homodimer and 4 µM calcein. Images were obtained within 30 minutes using a Nikon Diaphot microscope equipped with epifluorescence. The MetaMorph image analysis system (Universal Imaging Corporation, Philadelphia, PA) was used to quantify the number and/or area of cells showing green or red fluorescence.

For these experiments, ADDLs were present for 24 hours at a maximal 5 µM dose of total Aβ (i.e., total Aβ was never more than 5 µM in any ADDL experiment). Cell death, as shown by "false yellow staining", was almost completely confined to the stratum pyramidale (CA 3-4) and dentate gyrus (DG) suggesting strongly that principal neurons of the hippocampus (pyramidal and granule cells, respectively) are the targets of ADDL-induced toxicity. Furthermore, glia viability is unaffected by a 24 hour ADDL treatment of primary rat brain glia, as determined by trypan blue exclusion and MTT assay (Finch et al., unpublished). Dentate gyrus (DG) and CA3 regions were particularly sensitive and showed ADDL-evoked cell death in every culture obtained from animals aged P20 (weanlings) to P84 (young adult). Up to 40% of the cells in this region die following chronic exposure to ADDLs. The pattern of neuronal death was not identical to that observed for NMDA, which killed neurons in DG and CA1 but spared CA3.

Some cultures from hippocampal DG and CA3 regions of animals more than 20 days of age were treated with conventional preparations of fibrillar Aβ. Consistent with the non-diffusible nature of the fibrils, no cell death (yellow staining) was evident even at 20 µM. The staining pattern for live cells in this culture verified that the CA3/dentate gyrus region of the hippocampus was being examined. The extent of cell death observed after conventional Aβ treatment (i.e., fibrillar Aβ preparations) was indistinguishable from negative controls in which cultures were given medium, or medium with clusterin supplement. In typical controls, cell death was less than 5%. In fact, high viability in controls could be found even in cultures maintained several days beyond a typical experiment, which confirms that cell survival was not compromised by standard culture conditions.

A dose-response experiment was carried out to determine the potency of ADDLs in evoking cell death. Image analysis was used to quantify dead cell and live cell staining in fields containing the DG/CA3 areas. **Figure 5** illustrates the % dead cells verses ADDL concentration measured as initial amyloid β 1-42 concentration (nM). Because of the difficulties of quantifying brain slices, the results are not detailed enough to determine the EC50 with precision. However, as can be seen in **Figure 5,** even after 1000-fold dilution (~5 nM Aβ), ADDL-evoked cell death was more than 20%. Toxicity was observed even with 0.3 nM ADDLs. This contrasts with results obtained with conventionally aged Aβ, which is toxic to neurons in culture at about 20 to about 50 µM. These data show that ADDLs are effective at doses approximately 1,000-10,000-fold smaller than those used in fibrillar Aβ experiments.

These data from hippocampal slices thus confirm the ultratoxic nature of ADDLs. Furthermore, because ADDLs had to pass through the culture-support filter to cause cell death, the results validate that ADDLs are diffusible, consistent with their small oligomeric size. Also, the methods set forth herein can be employed as an assay for ADDL-mediated changes in cell viability. In particular, the assay can be carried out by coincubating or coadministering along with the ADDLs agents that potentially may increase or decrease ADDL formation and/or activity. Results obtained with such coincubation or coadministration can be compared to results obtained with inclusion of ADDLs alone.

### Example 7: MTT Oxidative Stress Toxicity Assay - PC12 Cells

This example sets forth an assay that can be employed to detect an early toxicity change in response to amyloid β oligomers.

For these experiments, PC12 cells were passaged at 4 x 10⁴ cells/well on a 96-well culture plate and grown for 24 hours in DMEM + 10% fetal calf serum + 1% S/P/F (streptomycin, penicillin, and fungizone). Plates were treated with 200 µg/mL poly-1-lysine for 2 hours prior to cell plating to enhance cell adhesion. One set of six wells was left untreated and fed with fresh media, while another set of wells was treated with the vehicle control (PBS containing 10% 0.01 N HCl, aged o/n at RT). Positive controls were treated with triton (1%) and Na Azide (0.1 %) in normal growth media. Amyloid β oligomers prepared as described in Example 1, or obtained upon coincubation with clusterin, with and without inhibitor compounds present, were added to the cells for 24 hours. After the 24 hour incubation, MTT (0.5 mg/mL) was added to the cells for 2.5 hours (11 µL of 5 mg/ml stock solubilized in PBS into 100 µL of media). Healthy cells reduce the MTT into a formazan blue colored product. After the incubation with MTT, the media was aspirated and 100 µL of 100% DMSO was added to lyse the cells and dissolve the blue crystals. The plate was incubated for 15 min at RT and read on a plate reader (ELISA) at 550 nm.

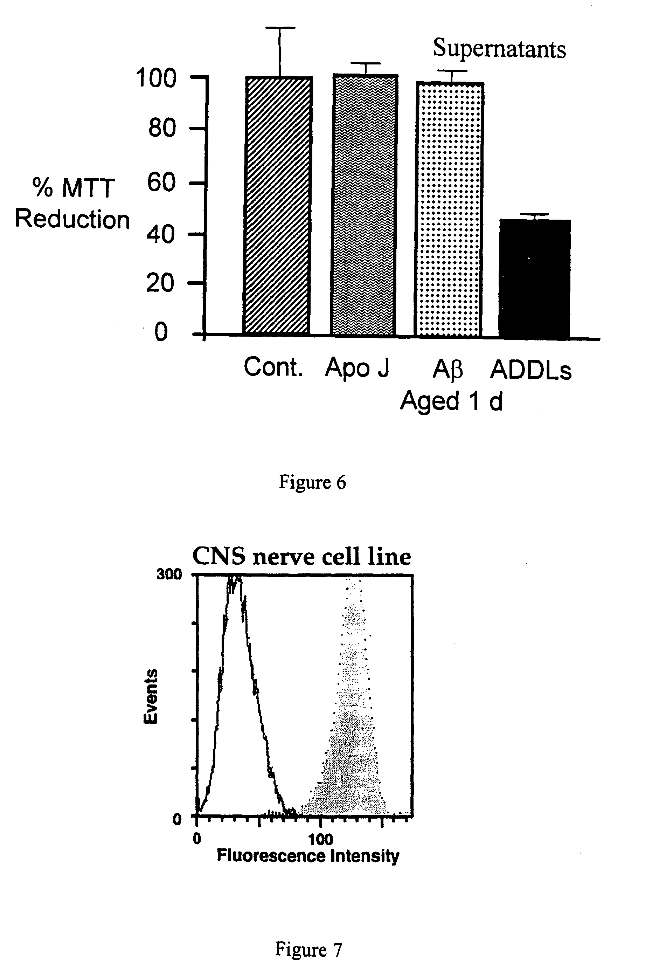

The results of one such experiment are depicted in **Figure 6**. As can be seen from this figure, control cells not exposed to ADDLs ("Cont."), cells exposed to clusterin alone ("Apo J"), and cells exposed to monomeric Aβ ("Aβ") show no cell toxicity. By contrast, cells exposed to amyloid β coaggregated with clusterin and aged one day ("Aβ:Apo J") show a decrease in MTT reduction, evidencing an early toxicity change. The lattermost amyloid preparations were confirmed by AFM to lack amyloid fibrils.

Results of this experiment thus confirm that that ADDL preparations obtained from coaggregation of Aβ mediated by clusterin have enhanced toxicity. Moreover, the results confirm that the PC 12 oxidative stress response can be employed as an assay to detect early cell changes due to ADDLs. The assay can be carried out by coincubating or coadministering along with the ADDLs agents that potentially may increase or decrease ADDL formation and/or activity. Results obtained with such coincubation or coadministration can be compared to results obtained with inclusion of ADDLs alone.

### Example 8: MTT Oxidative Stress Toxicity Assay - HN2 Cells

This example sets forth a further assay of ADDL-mediated cell changes. Namely, the MTT oxidative stress toxicity assay presented in the preceding example can be carried out with HN2 cells instead of PC 12 cells. Other appropriate cells similarly can be employed.

For this assay, HN2 cells were passaged at 4 x 10⁴ cells/well on a 96-well culture plate and grown for 24 hours in DMEM + 10% fetal calf serum + 1% S/P/F (streptomycin, penicillin, and fungizone). Plates were treated with 200 µg/mL poly 1-lysine for 2 hours prior to cell plating to enhance cell adhesion. The cells were differentiated for 24-48 hours with 5 µM retinoic acid and growth was further inhibited with 1% serum. One set of wells was left untreated and given fresh media. Another set of wells was treated with the vehicle control (0.2% DMSO). Positive controls were treated with triton (1%) and Na Azide (0.1%). Amyloid β oligomers prepared as described in example 1, with and without inhibitor compounds present, were added to the cells for 24 hours. After the 24 hour incubation, MTT (0.5 mg/mL) was added to the cells for 2.5 hours (11 µL of 5 mg/mL stock into 100 µL of media). After the incubation with MTT, the media was aspirated and 100 µL of 100% DMSO is added to lyse the cells and dissolve the blue crystals. The plate was incubated for 15 minutes at RT and read on a plate reader (ELISA) at 550 nm.

This assay similarly can be carried out by coincubating or coadministering along with the ADDLs agents that potentially may increase or decrease ADDL formation and/or activity. Results obtained with such coincubation or coadministration can be compared to results obtained with inclusion of ADDLs alone.

### Example 9: Cell Morphology by Phase Microscopy

This example sets forth yet another assay of ADDL-mediated cell changes - assay of cell morphology by phase microscopy.

For this assay, cultures were grown to low density (50-60% confluence). To initiate the experiment, the cells were serum-starved in F12 media for 1 hour. Cells were then incubated for 3 hours with amyloid β oligomers prepared as described in example 1, with and without inhibitor compounds added to the cells, for 24 hours. After 3 hours, cells were examined for morphological differences or fixed for immunofluorescence labeling. Samples were examined using the MetaMorph Image Analysis system and an MRI video camera (Universal Imaging, Inc.).

Results of such assays are presented in the examples which follow. In particular, the assay can be carried out by coincubating or coadministering along with the ADDLs agents that potentially may increase or decrease ADDL formation and/or activity. Results obtained with such coincubation or coadministration can be compared to results obtained with inclusion of ADDLs alone.

### Example 10: FACScan Assay for Binding of ADDLs to Cell Surfaces

Because cell surface receptors recently have been identified on glial cells for conventionally prepared Aβ (Yan et al., *Nature*, **382,** 685-691, 1996; El Khoury et al., *Nature*, **382,** 716-719, 1996), and because neuronal death at low ADDL doses suggested possible involvement of signaling mechanisms, experiments were undertaken to determine if specific cell surface binding sites on neurons exist for ADDLs.

For flow cytometry, cells were dissociated with 0.1% trypsin and plated at least overnight onto tissue culture plastic at low density. Cells were removed with cold phosphate buffered saline (PBS) /0.5 mM EDTA, washed three times and resuspended in ice-cold PBS to a final concentration of 500,000 cells/mL. Cells were incubated in cold PBS with amyloid β oligomers prepared as described in Example 1, except that 10% of the amyloid β is an amyloid β 1-42 analog containing biocytin at position 1 replacing aspartate. Oligomers with and without inhibitor compounds present were added to the cells for 24 hours. The cells were washed twice in cold PBS to remove free, unbound amyloid β oligomers, resuspended in a 1:1,000 dilution of avidin conjugated to fluorescein, and incubated for one hour at 4°C with gentle agitation. Alternately, amyloid β-specific antibodies and fluorescent secondary antibody were employed instead of avidin, eliminating the need to incorporate 10% of the biotinylated amyloid β analog. Namely, biotinylated 6E10 monoclonal antibody (1µL Senetec, Inc., St. Louis, Missouri) was added to the cell suspension and incubated for 30 minutes. Bound antibody was detected after pelleting cells and resuspending in 500 µL PBS, using FITC-conjugated streptavidin (1:500, Jackson Laboratories) for 30 minutes.

Cells were analyzed by a Becton-Dickenson Fluorescence Activated Cell Scanner (FACScan). 10,000 or 20,000 events typically were collected for both forward scatter (size) and fluorescence intensity, and the data were analyzed by Consort 30 software (Becton-Dickinson). Binding was quantified by multiplying mean fluorescence by total number of events, and subtracting value for background cell fluorescence in the presence of 6E10 and FITC.

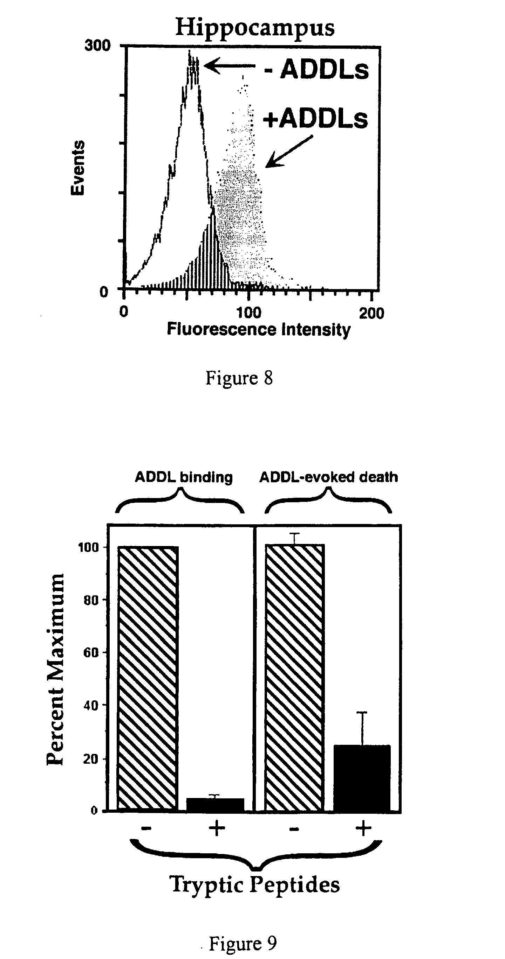

For these experiments, FACScan analysis was done to compare ADDL immunoreactivity in suspensions of log-phase yeast cells (a largely carbohydrate surface) and of the B103 CNS neuronal cell line (Schubert et al., *Nature,* **249**, 224-227, 1974). For B 103 cells, addition of ADDLs caused a major increase in cell associated fluorescence, as shown in **Figure 7**. Trypsin treatment of the B103 cells for 1 minute eliminated ADDL binding. In contrast, control yeast cells (data not shown) demonstrated no ADDL binding, verifying the selectivity of ADDLs for proteins present on the cell surface. Suspensions of hippocampal cells (trypsinized tissue followed by a two-hour metabolic recovery) also bound ADDLs, but with a reduced number of binding events compared with the B103 cells, as evidenced by the reduced fluorescence intensity of the labelled peak. This appears in **Figure 8** as a leftward shifting of the labelled peak.

These results thus suggest that the ADDLs exert their effects by binding to a specific cell surface receptor. In particular, the trypsin sensitivity of B103 cells showed that their ADDL binding sites were cell surface proteins and that binding was selective for a subset of particular domains within these proteins.

Moreover, the present assay can also be employed as an assay for ADDL-mediated cell binding. In particular, the assay can be carried out by coincubating or coadministering along with the ADDLs agents that potentially may increase or decrease ADDL formation and/or activity. Results obtained with such coincubation or coadministration can be compared to results obtained with inclusion of ADDLs alone.

### Example 11: Inhibition of ADDL Formation by Gossypol

This example sets forth the manner in which ADDL formation can be inhibited using, for instance, gossypol.

For these experiments, ADDLs were prepared as described in Example 1. Gossipol (Aldrich) was added to a concentration of 100 µM during the incubation of the Aβ protein to form ADDLs. The resulting preparation was assessed for neurotoxicity using the **LIVE/DEAD®** assay kit as previously described. The amount of cell death that occurred after 24 hours of exposure to the gossypol/ADDL preparation was less than 5%. This is comparable to the level of toxicity obtained for a corresponding DMSO control preparation (i.e., 6%), or a gossypol control preparation that did not contain any ADDLs (i.e., 4%).

These results thus confirm that compounds such as gossypol can be employed to inhibit ADDL formation.

### Example 12: Inhibition of ADDL Binding by Tryptic Peptides

Because B103 cell trypsinization was found to block subsequent ADDL binding, experiments were done as set forth in this example to test if tryptic fragments released from the cell surface retard ADDL binding activity.

Tryptic peptides were prepared using confluent B103 cells from four 100 mm dishes that were removed by trypsinization (0.025%, Life Technologies) for approximately 3 minutes. Trypsin-chymotrypsin inhibitor (Sigma, 0.5 mg/mL in Hank's Buffered Saline) was added, and cells were removed via centrifugation at 500 x g for 5 minutes. Supernatant (~12 mL) was concentrated to approximately 1.0 mL using a Centricon 3 filter (Amicon), and was frozen after the protein concentration was determined. For blocking experiments, sterile concentrated tryptic peptides (0.25 mg/mL) were added to the organotypic brain slice or to the suspended B103 cells in the FACs assay at the same time as the ADDLs were added.

In FACScan assays, tryptic peptides released into the culture media (0.25 mg/mL) inhibited ADDL binding by > 90% as shown in **Figure 9.** By comparison, control cells exposed to BSA, even at 100mg/mL, had no loss of binding. Tryptic peptides, if added after ADDLs were already attached to cells, did not significantly lower fluorescence intensities. This indicates that the peptides did not compromise the ability of the assay to quantify bound ADDLs. Besides blocking ADDL binding, the tryptic peptides also were antagonists of ADDL-evoked cell death. Namely, as shown in **Figure 9,** addition of tryptic peptides resulted in a 75% reduction in cell death, p < 0.002.

These data confirm that particular cell surface proteins mediate ADDL binding, and that solubilized tryptic peptides from the cell surface provide neuroprotective, ADDL-neutralizing activity. Moreover, the present assay can also be employed as an assay for agents that mediate ADDL cell binding or ADDL effects on cell activity. In particular, the assay can be carried out by coincubating or coadministering along with the ADDLs agents that potentially may increase or decrease ADDL formation and/or activity. Results obtained with such coincubation or coadministration can be compared to results obtained with inclusion of ADDLs alone. Moreover, addition of the agents before or after binding of the ADDLs to the cell surface can be compared to identify agents that impact such binding, or that act after binding has occurred.

### Example 13: Dose Response Curve for ADDL Cell Binding

This example sets forth dose response experiments done to determine whether ADDL binding to the cell surface is saturable. Such saturability would be expected if the ADDLs in fact interact with a particular cell surface receptor.

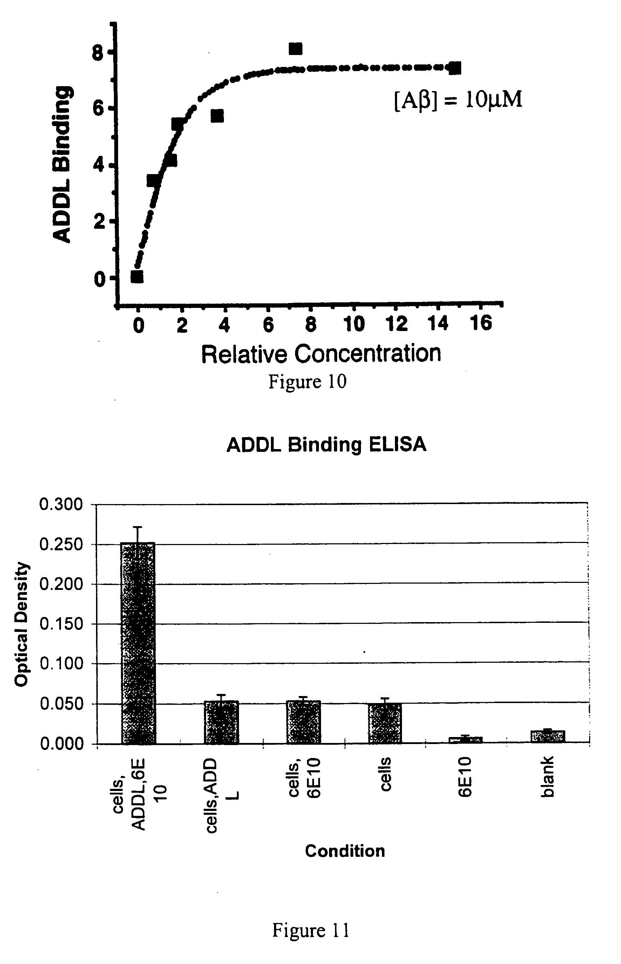

For these studies, B103 cells were incubated with increasing amounts of ADDLs and ADDL binding was quantitated by FACscan analysis. Results are presented in **Figure 10**. These results confirm that a distinct plateau is achieved for ADDL binding. Saturability of ADDL binding occurs at a relative Aβ 1-42 concentration (i.e., ADDL concentration relative to Aβ) of about 250 nm.

These results thus confirm that ADDL binding is saturable. Such saturability of ADDL binding, especially when considered with the results of the trypsin studies, validates that the ADDLs are acting through a particular cell surface receptor.

### Example 14: Cell-Based ELISA for ADDL Binding Activity

This example sets forth a cell-based assay, particularly a cell-based enzymelinked immunosorbent assay (ELISA) that can be employed to assess ADDL binding activity.

For these studies, 48 hours prior to conduct of the experiment, 2.5 x 10⁴ B103 cells present as a suspension in 100 µL DMEM were placed in each assay well of a 96-well microtiter plate and kept in an incubator at 37°C. 24 hours prior to the conduct of the experiment, ADDLs were prepared according to the method described in Example 1. To begin the assay, each microtiter plate well containing cells was treated with 50µL of fixative (3.7% formalin in DMEM) for 10 minutes at room temperature. This fixative/DMEM solution was removed and a second treatment with 50 µL formalin (no DMEM) was carried out for 15 minutes at room temperature. The fixative was removed and each well was washed twice with 100 µL phosphate buffered saline (PBS). 200 µL of a blocking agent (1% BSA in PBS) was added to each well and incubated at room temperature for 1 hour. After 2 washes with 100 µL PBS, 50 µL of ADDLs (previously diluted 1:10 in PBS), were added to the appropriate wells, or PBS alone as a control, and the resulting wells were incubated at 37°C for 1 hour. 3 washes with 100 µL PBS were carried out, and 50 µL biotinylated 6E10 (Senetek) diluted 1:1000 in 1% BSA/PBS was added to the appropriate wells. In other wells, PBS was added as a control. After incubation for 1 hour at room temperature on a rotator, the wells were washed 3 times with 50 µL PBS, and 50 µL of the ABC reagent (Elite ABC kit, Vector Labs) was added and incubated for 30 minutes at room temperature on the rotator. After washing 4 times with 50 µL PBS, 50 µL of ABTS substrate solution was added to each well and the plate was incubated in the dark at room temperature. The plate was analyzed for increasing absorption at 405 nm. Only when ADDLs, cells, and 6E10 were present was there a significant signal, as illustrated in **Figure 11.**

These results further confirm that a cell-based ELISA assay can be employed as an assay for ADDL-mediated cell binding. In particular, the assay can be carried out by coincubating or coadministering along with the ADDLs agents that potentially may increase or decrease ADDL formation and/or activity. Results obtained with such coincubation or coadministration can be compared to results obtained with inclusion of ADDLs alone.

### Example 15: Fyn kinase knockout protects against ADDL neurotoxicity

To investigate further the potential involvement of signal transduction in ADDL toxicity, the experiments in this example compared the impact of ADDLs on brain slices from isogenic *fyn* -/- and *fyn* +/+ animals. Fyn belongs to the Src-family of protein tyrosine kinases, which are central to multiple cellular signals and responses (Clarke et al., *Science,* **268**, 233-238). Fyn is of particular interest because it is upregulated in AD-afflicted neurons (Shirazi et al., *Neuroreport,* **4**, 435-437, 1993). It also appears to be activated by conventional Aβ preparations (Zhang et al., *Neurosci. Letts.,* **211**, 187-190, 1996) which subsequently have been shown to contain ADDLs by AFM. Fyn knockout mice, moreover, have reduced apoptosis in the developing hippocampus (Grant et al., *Science,* **258**, 1903-1910, 1992).

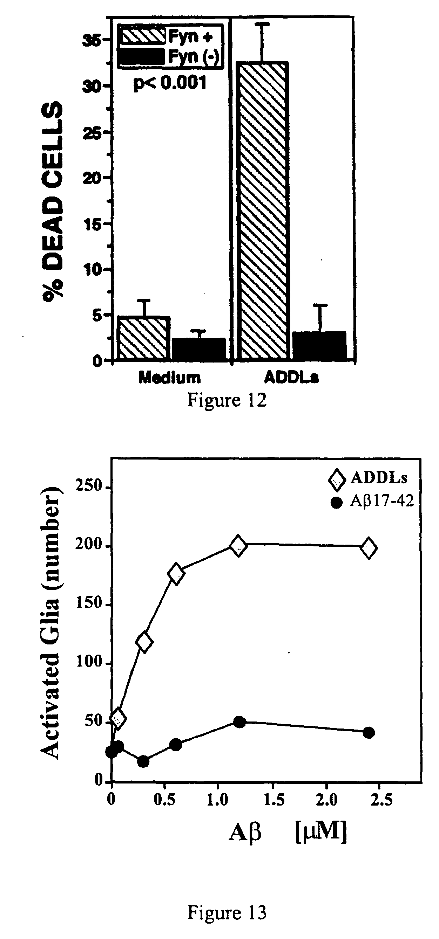

For these studies, Fyn knockout mice (Grant et al., *Science,* **258**, 1903-1910, 1992) were treated as described in the preceding examples, by comparing images of brain slices of mice either treated or not treated with ADDLs for 24 hours to determine dead cells in the DG and CA3 area. The quantitative comparison (presented in **Figure 12**) was obtained with error bars representing means +/-SEM for 4-7 slices.

In contrast to cultures from wild-type animals, cultures from *fyn* -/- animals showed negligible ADDL-evoked cell death, as shown in **Figure 12**. For ADDLs, the level of cell death in *fyn* +/+ slices was more than five times that in *fyn* -/- cultures. In *fyn* -/- cultures, cell death in the presence of ADDLs was at background level. The neuroprotective response was selective; hippocampal cell death evoked by NMDA receptor agonists (Bruce et al., *Exper. Neurol.,* **132**, 209-219, 1995; Vornov et al., *Neurochem.,* **56**, 996-1006, 1991) was unaffected (not shown). Analysis (ANOVA) using the Tukey multiple comparison gave a value of P < 0.001 for the ADDL *fyn* +/+ data compared to all other conditions.

These results confirm that loss of Fyn kinase protected DG and CA3 hippocampal regions from cell death induced by ADDLs. The results validate that ADDL toxicity is mediated by a mechanism blocked by knockout of Fyn protein tyrosine kinase. These results further suggest that neuroprotective benefits can be obtained by treatments that abrogate the activity of Fyn protein tyrosine kinase or the expression of the gene encoding Fyn protein kinase.

### Example 16: Astrocyte Activation Experiments

To investigate further the potential involvement of signal transduction in ADDL toxicity, the experiments in this example compared the impact on ADDLs on activation of astrocytes.

For these experiments, cortical astrocyte cultures were prepared from neonatal (1-2 day old) Sprague-Dawley rat pups by the method of Levison and McCarthy (Levison et al., In: Banker et al. (Eds.), *Culturing Nerve Cells,* MIT press, Cambridge, MA, , 309-36, 1991), as previously described (Hu et al., *J. Biol. Chem.,* **271,** 2543-2547, 1996). Briefly, cerebral cortex was dissected out, trypsinized, and cells were cultured in α-MEM (Gibco, BRL) containing 10% fetal bovine serum (Hyclone Laboratories Inc., Logan UT) and antibiotics (100 U/mL penicillin, 100 mg/mL streptomycin). After 11 days in culture, cells were trypsinized and replated into 100-mm plates at a density of ~6 x10⁵ cells/plate and grown until confluent (Hu et al., *J. Biol. Chem.,* **271,** 2543-2547, 1996).

Astrocytes were treated with ADDLs prepared according to Example 1, or with Aβ 17-42 (synthesized as per Lambert et al., *J. Neurosci. Res.,* **39**, 377-384, 1994; also commercially available). Treatment was done by trypsinizing confluent cultures of astrocytes and plating onto 60 mm tissue culture dishes at a density of 1 x 10⁶ cells/dish (e.g., for RNA analysis and ELISAs), into 4-well chamber slides at 5 x 10⁴ cells /well (e.g., for immunohistochemistry), or into 96-well plates at a density of 5 x 10⁴ cells/well (e.g., for NO assays). After 24 hours of incubation, the cells were washed twice with PBS to remove serum, and the cultures incubated in α-MEM containing N2 supplements for an additional 24 hours before addition of Aβ peptides or control buffer (i.e., buffer containing diluent).

Examination of astrocyte morphology was done by examining cells under a Nikon TMS inverted microscope equipped with a Javelin SmartCam camera, Sony video monitor and color video printer. Typically, four arbitrarily selected microscopic fields (20X magnification) were photographed for each experimental condition. Morphological activation was quantified from the photographs with NIH Image by counting the number of activated cells (defined as a cell with one or more processes at least one cell body in length) in the four fields.

The mRNA levels in the cultures was determined with use of Northern blots and slot blots. This was done by exposing cells to ADDLs or control buffer for 24 hours. After this time, the cells were washed twice with diethylpyrocarbonate (DEPC)-treated PBS, and total RNA was isolated by RNeasy purification mini-columns (Qiagen, Inc., Chatsworth, CA), as recommended by the manufacturer. Typical yields of RNA were 8 to 30 mg of total RNA per dish. For Northern blot analysis, 5 mg total RNA per sample was separated on an agarose-formaldehyde gel, transferred by capillary action to Hybond-N membrane (Amersham, Arlington Heights IL), and UV crosslinked. For slot blot analysis, 200 ng of total RNA per sample was blotted onto Duralon-UV membrane (Stratagene, La Jolla CA) under vacuum, and UV crosslinked. Confirmation of equivalent RNA loadings was done by ethidium bromide staining or by hybridization and normalization with a GAPDH probe.

Probes were generated by restriction enzyme digests of plasmids, and subsequent gel purification of the appropriate fragment. Namely, cDNA fragments were prepared by RT-PCR using total RNA from rat cortical astrocytes. RNA wasreverse transcribed with a Superscript II system (GIBCO/ BRL), and PCR was performed on a PTC-100 thermal controller (MJ Research Inc, Watertown, MA) using 35 cycles at the following settings: 52°C for 40 seconds; 72°C for 40 seconds; 96°C for 40 seconds. Primer pairs used to amplify a 447 bp fragment of rat IL-1β were: Forward: 5' GCACCTTCTTTCCCTTCATC 3' [SEQ ID NO:1]. Reverse: 5' TGCTGATGTACCAGTTGGGG 3' [SEQ ID NO:2]. Primer pairs used to amplify a 435 bp fragment of rat GFAP were: Forward: 5' CAGTCCTTGACCTGCGACC 3' [SEQ ID NO:3]. Reverse: 5' GCCTCACATCACATCCTTG 3' [SEQ ID NO:4]. PCR products were cloned into the pCR2.1 vector with the Invitrogen TA cloning kit, and constructs were verified by DNA sequencing. Probes were prepared by *Eco*RI digestion of the vector, followed by gel purification of the appropriate fragments. The plasmids were the rat iNOS cDNA plasmid pAstNOS-4, corresponding to the rat iNOS cDNA bases 3007-3943 (Galea et al., *J. Neurosci. Res.,* **37,** 406-414, 1994), and the rat GAPDH cDNA plasmid pTRI-GAPDH (Ambion, Inc., Austin TX).

The probes (25 ng) were labeled with ³²P-dCTP by using a Prime-a-Gene Random-Prime labeling kit (Promega, Madison WI) and separated from unincorporated nucleotides by use of push-columns (Stratagene). Hybridization was done under stringent conditions with QuikHyb solution (Stratagene), using the protocol recommended for stringent hybridization. Briefly, prehybridization was conducted at 68°C for about 30 to 60 minutes, and hybridization was at 68°C for about 60 minutes. Blots were then washed under stringent conditions and exposed to either autoradiography or phosphoimaging plate. Autoradiograms were scanned with a BioRad GS-670 laser scanner, and band density was quantified with Molecular Analyst v2.1 (BioRad, Hercules CA) image analysis software. Phosphoimages were captured on a Storm 840 system (Molecular Dynamics, Sunnyvale CA), and band density was quantified with Image Quant v1.1 (Molecular Dynamics) image analysis software.

For measurement of NO by nitrite assay, cells were incubated with Aβ peptides or control buffer for 48 hours, and then nitrite levels in the conditioned media were measured by the Griess reaction as previously described (Hu et al., *J. Biol. Chem.,* **271**, 2543-2547, 1996). When the NOS inhibitor N-nitro-L-arginine methylester (L-name) or the inactive D-name isomer were used, these agents were added to the cultures at the same time as the Aβ.

Results of these experiments are presented in **Figure 13**. As can be seen in this figure, glia activation increases when astrocytes are incubated with ADDLs, but not when astrocytes are incubated with Aβ 17-42.

These results confirm that ADDLs activate glial cells. It is possible that glial proteins may contribute to neural deficits, for instance, as occur in Alzheimer's Disease, and that some effects of ADDLs may actually be mediated indirectly by activation of glial cells. In particular, glial proteins may facilitate formation of ADDLs, or ADDL-mediated effects that occur downstream of receptor binding. Also, it is known that clusterin is upregulated in the brain of the Alzheimer's diseased subject, and clusterin is made at elevated levels only in glial cells that are activated. Based on this, activation of glial cells by a non-ADDL, non-amyloid stimulus could produce clusterin which in turn might lead to ADDLs, which in turn would damage neurons and cause further activation of glial cells.

Regardless of the mechanism, these results further suggest that neuroprotective benefits can be obtained by treatments that modulate (i.e., increase or decrease) ADDL-mediated glial cell activation. Further, the results suggest that blocking these effects on glial cells, apart from blocking the neuronal effects, may be beneficial.

### Example 17: LTP Assay-ADDLs Disrupt LTP

Long-term potentiation (LTP) is a classic paradigm for synaptic plasticity and a model for memory and learning, faculties that are selectively lost in early stage AD. This example sets forth experiments done to examine the effects of ADDLs on LTP, particularly medial perforant path-granule cell LTP.

**Injections of intact animals:** Mice were anesthesized with urethane and placed in a sterotaxic apparatus. Body temperature was maintained using a heated water jacket pad. The brain surface was exposed through holes in the skull. Bregma and lambda positions for injection into the middle molecular layer of hippocampus are 2 mm posterior to bregma, 1 mm lateral to the midline, and 1.2-1.5 mm ventral to the brain surface. Amyloid β oligomer injections were by nitrogen puff through ~ 10 nm diameter glass pipettes. Volumes of 20-50 nL of amyloid β oligomer solution (180 nM of amyloid β in phosphate buffered saline, PBS) were given over the course of an hour. Control mice received an equivalent volume of PBS alone. The animal was allowed to rest for varying time periods before the LTP stimulus is given (typically 60 minutes).

**LTP in injected animals:** Experiments follow the paradigm established by Routtenberg and colleagues for LTP in mice (Namgung et al., *Brain Research,* **689,** 85-92, 1995). Perforant path stimulation from the entorhinal cortex was used, with recording from the middle molecular layer and the cell body of the dentate gyrus. A population excitatory postsynaptic potential (pop-EPSP) and a population spike potential (pop-spike) were observed upon electrical stimulation. LTP could be induced in these responses by a stimulus of 3 trains of 400 Hz, 8 x 0.4 ms pulses/train (Namgung et al., *Brain Research,* **689,** 85-92, 1995). Recordings were taken for 2-3 hours after the stimulus (i.e., applied at time 0) to determine if LTP is retained. The animal was then sacrificed immediately, or was allowed to recover for either 1, 3, or 7 days and then sacrificed as above. The brain was cryoprotected with 30% sucrose, and then sectioned (30 µM) with a microtome. Some sections were placed on slides subbed with gelatin and others were analyzed using a free-floating protocol. Immunohistochemistry was used to monitor changes in GAP-43, in PKC subtypes, and in protein phosphorylation of tau (PHF-1), paxillin, and focal adhesion kinase. Wave forms were analyzed by machine as described previously (Colley et al., *J*. *Neurosci.,* **10,** 3353-3360, 1990). A 2-way ANOVA compares changes in spike amplitude between treated and untreated groups.

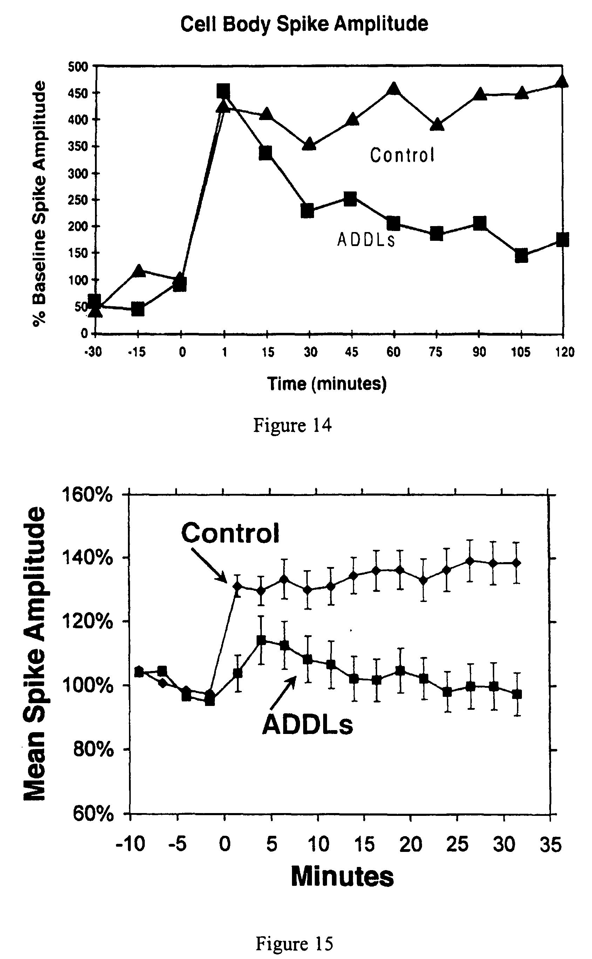

**Figure 14** illustrates the spike amplitude effect of ADDLs in whole animals. As can be clearly seen in this figure, ADDLs block the persistence phase of LTP induced by high frequency electrical stimuli applied to entorhinal cortex and measured as cell body spike amplitude in middle molecular layer of the dentate gyrus.

After the LTP experiment was performed, animals were allowed to recover for various times and then sacrificed using sodium pentobarbitol anesthetic and perfusion with 4% paraformaldehye. For viability studies, times of 3 hours, 24 hours, 3 days, and 7 days were used. The brain was cryoprotected with 30% sucrose and then sectioned (30 µM) with a microtome. Sections were placed on slides subbed with gelatin and stained initially with cresyl violet. Cell loss was measured by counting cell bodies in the dentate gyrus, CA3, CA1, and entorhinal cortex, and correlated with dose and time of exposure of ADDLs. The results of these experiments confirmed that no cell death occurred as of 24 hours following the LTP experiments.

Similarly, the LTP response was examined in hippocampal slices from young adult rats. As can be seen in **Figure 15**, incubation of rat hippocampal slices with ADDLs prevents LTP well before any overt signs of cell degeneration. Hippocampal slices (n=6) exposed to 500 nM ADDLs for 45 minutes prior showed no potentiation in the population spike 30 minutes after the tetanic stimulation (mean amplitude 99% +/- 7.6), despite a continuing capacity for action potentials. In contrast, LTP was readily induced in slices incubated with vehicle (n=6), with an amplitude of 138% +/-8.1 for the last 10 minutes; this value is comparable to that previously demonstrated in this age group (Trommer et al., *Exper. Neurol.,* **131**, 83-92, 1995). Although LTP was absent in ADDL-treated slices, their cells were competent to generate action potentials and showed no signs of degeneration.

These results validate that in both whole animals and tissue slices, the addition of ADDLs results in significant disruption of LTP in less than an hour, prior to any cell degeneration or killing. These experiments thus support that ADDLs exert very early effects, and interference with ADDL formation and/or activity thus can be employed to obtain a therapeutic effect prior to advancement of a disease, disorder, or condition (e.g., Alzheimer's disease) to a stage where cell death results. In other words, these results confirm that decreases in memory occur before neurons die. Interference prior to such cell death thus can be employed to reverse the progression, and potentially restore decreases in memory.

### Example 18: Early Effects of ADDLs in vivo

This example sets forth early effects of ADDLs *in vivo* and the manner in knowledge of such early effects can be manipulated.

The primary symptoms of Alzheimer's disease involve learning and memory deficits. However, the link between behavioral deficits and aggregated amyloid deposits has been difficult to establish. In transgenic mice, overexpressing mutant APP under the control of the platelet-derived growth factor promoter results in the deposition of large amounts of amyloid (Games et al., *Nature,* **373**, 523-527, 1995). By contrast, no behavioral deficits have been reported using this system. Other researchers (i.e., Nalbantoglu et al., *Nature,* **387,** 500-505, 1997 and Holcomb et al., *Nat. Med.,* **4,** 97-100, 1998) working with transgenic mice report observing significant behavioral and cognitive deficits that occur well before any significant deposits of aggregated amyloid are observed. These behavioral and cognitive defects include failure to long-term potentiate (Nalbantoglu et al., *supra*). These models collectively suggest that non-deposited forms of amyloid are responsible for the early cognitive and behavioral deficits that occur as a result of induced neuronal malfunction. It is consistent with these models that the novel ADDLs described herein are this non-deposited form of amyloid causing the early cognitive and behavioral defects. In view of this, ADDL modulating compounds according to the invention can be employed in the treatment and/or prevention of these early cognitive and behavioural deficits resulting from ADDL-induced neuronal malfunction, or ADDLs themselves can be applied, for instance, in animal models, to study such induced neuronal malfunction.

Similarly, in elderly humans, cognitive decline and focal memory deficits can occur well before a diagnosis of probable stage I Alzheimer's disease is made (Linn et al., *Arch. Neurol,* **52**, 485-490, 1995). These focal memory deficits may result from induced abberant signaling in neurons, rather than cell death. Other functions, such as higher order writing skills (Snowdon et al., *JAMA,* **275,** 528-532, 1996) also may be affected by abberant neuronal function that occurs long before cell death. It is consistent with what is known regarding these defects, and the information regarding ADDLs provided herein, that ADDLs induce these defects in a manner similar to compromised LTP function such as is induced by ADDLs. Along these lines, ADDL modulating compounds according to the invention can be employed in the treatment and/or prevention of these early cognitive decline and focal memory deficits, and impairment of higher order writing skills, resulting from ADDL formation or activity, or ADDLs themselves can be applied, for instance, in animal models, to study such induced defects. In particular, such studies can be conducted such as is known to those skilled in the art, for instance by comparing treated or placebo-treated age-matched subjects.

## Claims

1. An isolated and Homogeneous, soluble, globular, nou-fibrillar amyloid a oligomeric structure comprising 4 to 12 amyloid β1-42 proteins, wherein the structure exhibits neurotoxicity and does not contain amyloid β1-40 protein.

2. The oligomeric structure according to Claim 1 wherein the oligomeric structure comprises an oligomeric form selected from the group consisting of tetramer, pentamer, and hexamer aggregates of amyloid β1-42 protein.

3. The oligomeric structure according to Claim 1 or 2 wherein the oligomeric structure has a molecular weight of from 26 kD to 28 kD as determined by non-denaturing gel electrophoresis.

4. The oligomeric structure according to any of Claims 1 to 3 wherein the oligomeric structure has a molecular weight of from 22 kD to 24 kD or from 18 kD to 19 kD as determined by electrophoresis on a 15% SDS-polyacrylamide gel.

5. The oligomeric structure according to any of claims 1 to 4 wherein the oligomeric structure comprises globules of dimensions of from 4.7 nm to 6.2 nm as measured by atomic force microscopy.

6. The oligomeric structure according to any of Claims 1 to 5 wherein the oligomeric structure comprises globules of dimensions of from 4.9 nm to 5.4 nm as measured by atomic force microscopy.

7. The oligomeric structure according to any of Claims 1 to 5 wherein the oligomeric structure comprises globules of dimensions of from 5.7 nm to 6.2 nm as measured by atomic force microscopy.

8. The oligomeric structure according to any of Claims 1 to 5 wherein from 40% to 75% of the oligomeric structure comprises globules of dimensions of from 4.9 nm to 5.4 nm, and dimensions of from 5.7 nm to 62 nm, as measured by atomic force microscopy.

9. A method for identifying test compounds that block the neurotoxicity of the oligomeric structure of any of Claims 1 to 8, the method comprising:
(a) contacting separate cultures of neuronal cells with the oligomeric structure either in the presence or absence of contacting with the test compound;
(b) measuring the proportion of viable cells in each culture; and
(c) comparing the proportion of viable cells in each culture, with compounds that block the neurotoxicity of the oligomeric structure being identified as resulting in an increased proportion of viable cells in said culture as compared to the corresponding culture contacted with the oligomeric structure in the absence of the test compound.

10. A method for identifying test compounds that block binding to a cell surface protein of the oligomeric structure of any of Claims 1 to 8, the method comprising:
(a) contacting separate cultures of neuronal cells with the oligomeric structure either in the presence or absence of contacting with the test compound;
(b) adding a reagent that binds to the oligomeric structure, the reagent being fluorescent;
(c) analyzing said separate cell cultures by fluorescence-activated cell sorting; and
(d) comparing the fluorescence of the cultures, with compounds that block binding to a cell surface protein of the oligomeric structure being identified as resulting in a reduced fluorescence of the culture as compared to the corresponding culture contacted with the oligomeric structure in the absence of the test compound.

11. A method for identifying compounds that block binding to a cell surface protein of the oligomeric structure of any of Claims 1 to 8, the method comprising:
(a) forming the oligomeric structure from amyloid β1-42 protein such that it becomes a labeled oligomeric structure comprising a binding moiety capable of binding a fluorescent reagent;
(b) contacting separate cultures of neuronal cells with the labeled oligomeric structure either in the presence or absence of contacting with the test compound;
(c) adding a fluorescent reagent that binds to the oligomeric structure;
(d) analyzing the separate cell cultures by fluorescence-activated cell sorting; and
(e) comparing the fluorescence of the cultures, with compounds that block binding to a cell surface protein of the oligomeric structure being identified as resulting in a reduced fluorescence of the culture as compared to the corresponding culture contacted with the oligomeric structure in the absence of the test compound.

12. A method for identifying compounds that block formation or binding to a cell surface protein of the oligomeric structure of any of Claims 1 to 8, the method comprising:
(a) preparing separate samples of amyloid β1-42 protein that either have or have not been mixed with the test compound;
(b) forming the oligomeric structure in the separate samples;
(c) contacting separate cultures of neuronal cells with the separate samples;
(d) adding a reagent that binds to the oligomeric structure, the reagent being fluorescent;
(e) analyzing the separate cell cultures by fluorescence-activated cell sorting; and
(f) comparing the fluorescence of the cultures, with compounds that block formation or binding to a cell surface protein of the oligomeric structure being identified as resulting in a reduced fluorescence of the culture as compared to the corresponding culture contacted with the oligomeric structure in the absence of the test compound.

13. A method for identifying compounds that block formation or binding to a cell surface protein of the oligomeric structure of any of Claims 1 to 8, the method comprising:
(a) preparing separate samples of amyloid β1-42 protein that either have or have not been mixed with the test compound;
(b) forming the oligomeric structure in the separate samples such that it becomes a labeled oligomeric structure comprising a binding moiety capable of binding a fluorescent reagent in each of the separate samples;
(c) contacting separate cultures of neuronal cells with the separate samples;
(d) adding a fluorescent reagent that binds to the oligomeric structure;
(e) analyzing the separate cell cultures by fluorescence-activated cell sorting; and
(f) comparing the fluorescence of the cultures, with compounds that block formation or binding to a cell surface protein of the oligomeric structure being identified as resulting in a reduced fluorescence of the culture as compared to the corresponding culture contacted with the oligomeric structure in the absence of the test compound.

14. The method of Claim 12 or 13, wherein the fluorescence of the cultures further is compared with the fluorescence of cultures that have been treated in the same fashion except that instead of adding or not adding test compound prior to formation of the oligomeric structure, the test compound either is or is not added after formation of the oligomeric structure,
with compounds that block formation of the oligomeric structure being identified as resulting in a reduced fluorescence of the culture as compared to the corresponding culture contacted with the oligomeric structure in the absence of the test compound, only when the compound is added prior to oligomeric structure, and
compounds that block binding to a cell surface protein of the oligomeric structure being identified as resulting in a reduced fluorescence of the culture as compared to the corresponding culture contacted with the oligomeric structure in the absence of the test compound, when the compound is added either prior to or after oligomeric structure.

15. A method of detecting binding to a cell surface protein of the oligomeric structure of any of Claims 1 to 8, the method comprising:
(a) forming the oligomeric structure from amyloid β1-42 protein;
(b) contacting a culture of neuronal cells with the oligomeric structure;
(c) adding an antibody that binds said oligomeric structure, the antibody including a conjugating moiety;
(d) washing away unbound antibody;
(f) linking an enzyme to the antibody bound to the oligomeric structure by means of the conjugating moiety;
(g) adding a colorless substrate that is cleaved by the enzyme to yield a color change; and
(h) determining the color change as a measure of binding to a cell surface protein of the oligomeric structure.

16. A method for identifying compounds that block binding to a cell surface protein of the oligomeric structure of any of Claims 1 to 8, the method comprising:
(a) preparing separate samples of amyloid β1-42 protein that either have or have not been mixed with the test compound;
(b) forming the oligomeric structure in the separate samples;
(c) contacting separate cultures of neuronal cells with the separate samples;
(d) adding an antibody that binds the oligomeric structure, the antibody including a conjugating moiety;
(e) washing away unbound antibody;
(f) linking an enzyme to the antibody bound to the oligomeric structure by means of the said conjugating moiety;
(g) adding a colorless substrate that is cleaved by the enzyme to yield a color change; and
(h) comparing the color change produced by each of the separate samples, with compounds that block formation or binding to a cell surface protein of the oligomeric structure being identified as resulting in a reduced color change produced by the culture as compared to the corresponding culture contacted with the oligomeric structure in the absence of the test compound.

17. The method of Claim 16, wherein the color change produced by the cultures further is compared with the color change produced by cultures that have been treated in the same fashion except that instead of adding or not adding test compound prior to formation of the oligomeric structure, the [said] test compound either is or is not added after formation of the oligomeric structure,
with compounds that block formation of the oligomeric structure being identified as resulting in a reduced color change produced by the culture as compared to the corresponding culture contacted with the oligomeric structure in the absence of the test compound, only when the compound is added prior to oligomeric structure, and
compounds that block receptor binding of the oligomeric structure being identified as resulting in a reduced color change produced by the culture as compared to the corresponding culture contacted with the oligomeric structure in the absence of the test compound, when the compound is added either prior to or after oligomeric structure.

18. A method for identifying compounds that block formation of the oligomeric structure of any of Claims 1 to 8, the method comprising:
(a) preparing separate samples of amyloid β1-42 protein that either have or have not been mixed with the test compound;
(b) forming the oligomeric structure in the separate samples;
(c) assessing whether any protein assemblies have formed in the separate samples using a method selected from the group consisting of electrophoresis, immunorecognition, and atomic force microscopy; and
(d) comparing the formation of the protein assemblies in the separate samples, with compounds that block formation of the oligomeric structure being identified as resulting in decreased formation of the oligomeric structure in the sample. as compared with a sample in which the oligomeric structure is formed in the absence of the test compound.

19. A method of preparing the oligomeric structure according to any of claims 1 to 8, wherein the method comprises:
(a) obtaining a solution of monomeric amyloid β1-42 protein, said amyloid β1-42 protein being capable of forming the oligomeric structure;
(b) diluting the protein solution into an appropriate media to a final concentration of about 5 nM to about 500 µM;
(c) incubating the media resulting from step (b) at about 4°C for about 2 hours to about 48 hours;
(c) centrifuging the solution at about 14,000 g at about 4°C; and
(d) recovering the supernatant resulting from the centrifugation as containing the amyloid β1-42 oligomeric structure.

20. The method of Claim 19, wherein the method comprises incubating the media resulting from step (b) at about 4°C in the presence of clusterin.

21. An isolated soluble, globular, non-fibrillar amyloid β oligomeric structure prepared according to Claim 19 or 20.

22. The use of the oligomeric structure according to any of Claims 1 to 8 to alter the long-term potentiation response of a nerve cell, comprising contacting the cell in vitro with the oligomeric structure.

23. The use of the oligomeric structure according to any of Claims 1 to 8 to cause morphological change of a nerve cell comprising contacting the cell in vitro with the oligomeric structure.

24. The use according to Claim 23, wherein, the morphological change includes an effect selected from the group consisting of cell killing, altering Fyn kinase activity, altering Fyn kinase subcellular localization, and altering mRNA levels for proteins including interleukin-1, inducible nitric oxide synthase, Apo E, Apo J, and α1-antichymotrypsin.

25. The use of the oligomeric structure according to any of Claims 1 to 8 to cause astrocyte activation, comprising contacting an astrocyte in vitro with the oligomeric structure.

26. The use of the oligomeric structure according to any of Claims 1 to 8 to identify test compounds that block the neurotoxicity of the oligomeric structure, comprising contacting a nerve cell in vitro with the oligomeric structure and the test compound.

27. The use of the oligomeric structure according to any of Claims 1 to 8 to identify test compounds that block the binding to a cell surface protein of the oligomeric structure, comprising contacting a nerve cell in vitro with the oligomeric structure and the test compound.

28. The use of the oligomeric structure according to any of Claims 1 to 8 to identify test compounds that block the formation of the oligomeric structure, comprising contacting amyloid β1-42 protein with the test compound during incubation to form the oligomeric structure.

29. The use of the oligomeric structure according to any of Claims 1 to 8 to cause ADDL-induced aberrant neuronal signaling of a nerve cell, comprising contacting the cell with the oligomeric structure.

## Patentansprüche

1. Isolierte, oligomere Struktur, die aus einem homogenen, lösbaren, kugelförmigen und nicht faserigen Amyloid besteht, das 4-12 Amyloid-β1-42-Proteine aufweist,
**dadurch gekennzeichnet,**
**dass** die Struktur eine Nervenschädigung vermeidet und kein Amyloid-β1-40-Protein aufweist.

2. Oligomere Struktur nach Anspruch 1,
**dadurch gekennzeichnet,**
**dass** sie eine oligomere Form aufweist, die aus der Gruppe ausgewählt ist, die tetramere, pentamere und hexamere Gruppierungen des Amyloid-β1-42-Proteins enthält.

3. Oligomere Struktur nach Anspruch 1 oder 2,
**dadurch gekennzeichnet,**
**dass** sie ein Molekulargewicht von 26-28kD aufweist, das durch eine Elektrophorese eines nicht denaturierenden Gels festgelegt wird.

4. Oligomere Struktur nach einem der Ansprüche 1 bis 3,
**dadurch gekennzeichnet,**
**dass** sie ein Molekulargewicht von 22-24kD oder von 18-19kD aufweist, das durch eine Elektrophorese eines 15%igen SDS-Polyakrylamid-Gels festgelegt wird.

5. Oligomere Struktur nach einem der Ansprüche 1 bis 4,
**dadurch gekennzeichnet,**
**dass** sie Kugeln mit einem Durchmesser von 4,7-6,2nm aufweist, der mittels der Atommikroskopie gemessen wird.

6. Oligomere Struktur nach einem der Ansprüche 1 bis 5,
**dadurch gekennzeichnet,**
**dass** sie Kugeln mit einem Durchmesser von 4,9-5,4nm aufweist, der mittels der Atommikroskopie gemessen wird.

7. Oligomere Struktur nach einem der Ansprüche 1 bis 5,
**dadurch gekennzeichnet,**
**dass** sie Kugeln mit einem Durchmesser von 5,7-6,2nm aufweist, der mittels der Atommikroskopie gemessen wird.

8. Oligomere Struktur nach einem der Ansprüche 1 bis 5,
**dadurch gekennzeichnet,**
**dass** 40-75% der oligomeren Struktur Kugeln mit einem Durchmesser von 4,9-5,4nm und mit einem Durchmesser von 5,7-6,2nm aufweist, die mittels der Atommikroskopie gemessen werden.

9. Verfahren zur Identifizierung von Testverbindungen, die eine Nervenschädigung der oligomeren Struktur nach einem der Ansprüche 1 bis 8 verhindern, **gekennzeichnet durch** folgende Schritte:
(a) getrennte Kulturen von Nervenzellen werden mit der oligomeren Struktur entweder zusammen mit der oder ohne die Testverbindung zusammengebracht,
(b) der Anteil der lebensfähigen Zellen wird gemessen, und
(c) der Anteil der lebensfähigen Zellen in jeder Kultur wird mit Verbindungen verglichen, die die Nervenschädigung der oligomeren Struktur vermeiden, wobei diese Verbindungen **dadurch** identifiziert werden, dass sich ein erhöhter Anteil von lebensfähigen Zellen in der betreffenden Kultur im Vergleich mit der entsprechenden Kultur ergibt, die mit der oligomeren Struktur in Abwesenheit der Testverbindung zusammengebracht worden ist.

10. Verfahren zur Identifizierung von Testverbindungen, die eine Bindung an ein Zelloberflächenprotein der oligomeren Struktur nach einem der Ansprüche 1 bis 8 verhindern,
**gekennzeichnet durch** folgende Schritte:
(a) getrennte Kulturen von Nervenzellen werden mit der oligomeren Struktur entweder zusammen mit der oder ohne die Testverbindung zusammengebracht,
(b) ein fluoreszierendes Reagens wird zugefügt, das sich an die oligomere Struktur bindet,
(c) die getrennten Zellstrukturen werden **durch** fluoreszenzaktivierte Zellsortierung analysiert, und
(d) die Fluoreszenz der Kulturen wird mit Verbindungen verglichen, die die Nervenschädigung der oligomeren Struktur vermeiden, wobei diese Verbindungen **dadurch** identifiziert werden, dass sich eine verminderte Fluoreszenz der betreffenden Kultur im Vergleich mit der entsprechenden Kultur ergibt, die mit der oligomeren Struktur in Abwesenheit der Testverbindung zusammengebracht worden ist.

11. Verfahren zur Identifizierung von Testverbindungen, die eine Bindung an ein Zelloberflächenprotein der oligomeren Struktur nach einem der Ansprüche 1 bis 8 verhindern,
**gekennzeichnet durch** folgende Schritte:
(a) die oligomere Struktur wird aus Amyloid-β1-42-Proteinen derart gebildet, dass sie zu einer markierten, oligomeren Struktur wird, die eine Bindungsverunreinigung aufweist, die ein fluoreszierendes Reagens binden kann,
(b) getrennte Kulturen aus Nervenzellen werden mit der markierten, oligomeren Struktur entweder unter Anwesenheit oder Abwesenheit der Testverbindung zusammengebracht,
(c) ein fluoreszierendes Reagens wird zugefügt, das sich an die oligomere Struktur bindet,
(d) die getrennten Zellstrukturen werden **durch** fluoreszenzaktivierte Zellsortierung analysiert, und
(e) die Fluoreszenz der Kulturen wird mit Verbindungen verglichen, die die Nervenschädigung der oligomeren Struktur vermeiden, wobei diese Verbindungen **dadurch** identifiziert werden, dass sich eine verminderte Fluoreszenz der betreffenden Kultur im Vergleich mit der entsprechenden Kultur ergibt, die mit der oligomeren Struktur in Abwesenheit der Testverbindung zusammengebracht worden ist.

12. Verfahren zur Identifizierung von Testverbindungen, die eine Bildung einer oligomeren Struktur oder Bindung an ein Zelloberflächenprotein der oligomeren Struktur nach einem der Ansprüche 1 bis 8 verhindern,
**gekennzeichnet durch** folgende Schritte:
(a) getrennte Proben aus Amyloid-β1-42-Proteinen werden zubereitet, die entweder mit der Testverbindung gemischt oder mit dieser nicht gemischt worden sind,
(b) die oligomere Struktur wird in den getrennten Proben gebildet,
(c) die getrennten Kulturen aus Nervenzellen werden mit den getrennten Proben zusammengebracht,
(d) ein fluoreszierendes Reagens wird zugefügt, das sich an die oligomere Struktur bindet,
(e) die getrennten Zellstrukturen werden **durch** fluoreszenzaktivierte Zellsortierung analysiert, und
(f) die Fluoreszenz der Kulturen wird mit Verbindungen verglichen, die die Bildung der oligomeren Struktur oder die Bindung an ein Zelloberflächenprotein der oligomeren Struktur vermeiden, wobei diese Verbindungen **dadurch** identifiziert werden, dass sich eine verminderte Fluoreszenz der betreffenden Kultur im Vergleich mit der entsprechenden Kultur ergibt, die mit der oligomeren Struktur in Abwesenheit der Testverbindung zusammengebracht worden ist.

13. Verfahren zur Identifizierung von Testverbindungen, die eine Bildung einer oligomeren Struktur oder Bindung an ein Zelloberflächenprotein der oligomeren Struktur nach einem der Ansprüche 1 bis 8 verhindern,
**gekennzeichnet durch** folgende Schritte:
(a) getrennte Proben aus Amyloid-β1-42-Proteinen werden zubereitet, die entweder mit der Testverbindung gemischt oder mit dieser nicht gemischt worden sind,
(b) die oligomere Struktur wird in den getrennten Proben derart gebildet, dass sie zu einer markierten, oligomeren Struktur wird, die eine Bindungsverunreinigung aufweist, die ein fluoreszierendes Reagens in jeder der getrennten Proben binden kann,
(c) die getrennten Kulturen aus Nervenzellen werden mit den getrennten Proben zusammengebracht,
(d) ein fluoreszierendes Reagens wird zugefügt, das sich an die oligomere Struktur bindet,
(e) die getrennten Zellstrukturen werden **durch** fluoreszenzaktivierte Zellsortierung analysiert, und
(f) die Fluoreszenz der Kulturen wird mit Verbindungen verglichen, die die Bildung der oligomeren Struktur oder die Bindung an ein Zelloberflächenprotein der oligomeren Struktur vermeiden, wobei diese Verbindungen **dadurch** identifiziert werden, dass sich eine verminderte Fluoreszenz der betreffenden Kultur im Vergleich mit der entsprechenden Kultur ergibt, die mit der oligomeren Struktur in Abwesenheit der Testverbindung zusammengebracht worden ist.

14. Verfahren nach Anspruch 12 oder 13,
**dadurch gekennzeichnet,**
**dass** die Fluoreszenz der Kulturen ferner mit der Fluoreszenz derjenigen Kulturen verglichen wird, die in derselben Weise, jedoch mit der Ausnahme behandelt worden sind, dass anstatt des Zufügens oder Nichtzufügens der Testverbindung vor der Bildung der oligomeren Struktur die Testverbindung nach der Bildung der oligomeren Struktur entweder zugefügt oder nicht zugefügt wird, und zwar
- mit Verbindungen, die die Bildung der oligomeren Struktur vermeiden, wobei diese Verbindungen **dadurch** identifiziert werden, dass sich eine verminderte Fluoreszenz der betreffenden Kultur im Vergleich mit der entsprechenden Kultur ergibt, die mit der oligomeren Struktur in Abwesenheit
- der Testverbindung zusammengebracht worden ist, aber nur dann, wenn die Verbindung vor der oligomeren Struktur zugefügt worden ist, und
- mit Verbindungen, die die Bindung an ein Zelloberflächenprotein der oligomeren Struktur vermeiden, wobei diese Verbindungen **dadurch** identifiziert werden, dass sich eine verminderte Fluoreszenz der betreffenden Kultur im Vergleich mit der entsprechenden Kultur ergibt, die mit der oligomeren Struktur in Abwesenheit der Testverbindung zusammengebracht worden ist, aber nur dann, wenn die Verbindung entweder vor oder nach der oligomeren Struktur zugefügt worden ist.

15. Verfahren zur Feststellung der Bindung an ein Zelloberflächenprotein der oligomeren Struktur nach einem der Ansprüche 1 bis 8,
**gekennzeichnet durch** folgende Schritte:
(a) die oligomere Struktur wird von einem Amyloid-β1-42-Protein gebildet,
(b) eine Kultur aus Nervenzellen wird mit der oligomeren Struktur zusammengebracht,
(c) ein Antikörper wird zugefügt, der die oligomere Struktur bindet und der eine korrespondierende Verunreinigung aufweist,
(d) der ungebundene Antikörper wird weggewaschen,
(e) ein Enzym wird mit dem Antikörper verbunden, der an die oligomere Struktur mittels der korrespondierenden Verunreinigung gebunden ist,
(f) ein farbloses Substrat wird zugefügt, das **durch** das Enzym gespaltet wird, um eine Farbänderung hervorzurufen, und
(g) die Farbänderung wird als Messung der Bindung an ein Zelloberflächenprotein der oligomeren Struktur festgestellt.

16. Verfahren zur Identifizierung von Verbindungen, die eine Bindung an ein Zelloberflächenprotein der oligomeren Struktur nach einem der Ansprüche 1 bis 8 verhindern, **gekennzeichnet durch** folgende Schritte:
(a) getrennte Proben aus Amyloid-β1-42-Proteinen werden zubereitet, die entweder mit der Testverbindung gemischt oder mit dieser nicht gemischt worden sind,
(b) die oligomere Struktur wird in den getrennten Proben gebildet,
(c) die getrennten Kulturen aus Nervenzellen werden mit den getrennten Proben zusammengebracht,
(d) ein die oligomere Struktur bindender Antikörper wird zugefügt, der eine korrespondierende Verunreinigung aufweist,
(e) der ungebundene Antikörper wird weggewaschen,
(f) ein Enzym wird mit dem Antikörper verbunden, der an die oligomere Struktur mittels der korrespondierenden Verunreinigung gebunden ist,
(g) ein farbloses Substrat wird zugefügt, das **durch** das Enzym gespaltet wird, um eine Farbänderung hervorzurufen, und
(h) die **durch** jede der getrennten Proben erzeugte Farbänderung wird mit Verbindungen verglichen, die die Bildung der oligomeren Struktur oder die Bindung an ein Zelloberflächenprotein der oligomeren Struktur vermeiden, wobei diese Verbindungen **dadurch** identifiziert werden, dass sich eine verminderte, **durch** die Kultur erzeugte Farbänderung im Vergleich mit der entsprechenden Kultur ergibt, die mit der oligomeren Struktur in Abwesenheit der Testverbindung zusammengebracht worden ist.

17. Verfahren nach Anspruch 16,
**dadurch gekennzeichnet,**
**dass** die durch die Kulturen erzeugte Farbänderung ferner mit derjenigen durch die Kulturen erzeugten Farbänderung verglichen wird, die in derselben Weise, jedoch mit der Ausnahme behandelt worden sind, dass anstatt des Zufügens oder Nichtzufügens der Testverbindung vor der Bildung der oligomeren Struktur die Testverbindung nach der Bildung der oligomeren Struktur entweder zugefügt oder nicht zugefügt wird, und zwar
- mit Verbindungen, die die Bildung der oligomeren Struktur vermeiden, wobei diese Verbindungen **dadurch** identifiziert werden, dass sich eine verminderte, durch die Kultur erzeugte Farbänderung im Vergleich mit der entsprechenden Kultur ergibt, die mit der oligomeren Struktur in Abwesenheit der Testverbindung zusammengebracht worden ist, aber nur dann, wenn die Verbindung vor der oligomeren Struktur zugefügt worden ist, und
- mit Verbindungen, die die Bindung der oligomeren Struktur vermeiden, wobei diese Verbindungen **dadurch** identifiziert werden, dass sich eine verminderte, durch die Kultur erzeugte Farbänderung im Vergleich mit der entsprechenden Kultur ergibt, die mit der oligomeren Struktur in Abwesenheit der Testverbindung zusammengebracht worden ist, aber nur dann, wenn die Verbindung entweder vor oder nach der oligomeren Struktur zugefügt worden ist.

18. Verfahren zur Identifizierung von Verbindungen, die eine Bildung der oligomeren Struktur nach einem der Ansprüche 1 bis 8 verhindern,
**gekennzeichnet durch** folgende Schritte:
(a) getrennte Proben aus Amyloid-β1-42-Proteinen werden zubereitet, die entweder mit der Testverbindung gemischt oder mit dieser nicht gemischt worden sind,
(b) die oligomere Struktur wird in den getrennten Proben gebildet,
(c) es wird ermittelt, ob eine Proteinanordnung in den getrennten Proben gebildet worden ist, wobei ein Verfahren verwendet wird, das aus der Gruppe ausgewählt ist, die aus der Elektrophorese, der Immunerkennung und der Atommikroskopie besteht, und
(d) die Bildung der Proteinanordnungen in den getrennten Proben wird mit Verbindungen verglichen, die die Bildung der oligomeren Struktur vermeiden, wobei diese Verbindungen **dadurch** identifiziert werden, dass sich eine verminderte Bildung der oligomeren Struktur in der Probe im Vergleich mit einer Probe ergibt, in der die oligomere Struktur in Abwesenheit der Testverbindung gebildet ist.

19. Verfahren zum Präparieren der oligomeren Struktur nach einem der Ansprüche 1 bis 8,
**gekennzeichnet durch** folgende Schritte:
(a) eine Lösung aus monomerem Amyloid-β1-42-Protein wird hergestellt, die die oligomere Struktur bilden kann,
(b) die Proteinlösung wird mit einem geeigneten Medium auf eine Endkonzentration von etwa 5nM bis etwa 500 µM verdünnt,
(c) das sich aus dem Schritt (b) ergebende Medium wird bei etwa 4°C für etwa 2-48 Stunden bebrütet,
(d) die Lösung wird bei etwa 14,000g und bei etwa 4°C zentrifugiert und
(e) die Schwimmdecke, die sich aus der Zentrifugation als oligomere Amyloid-β1-42-Struktur ergibt, wird abgeschöpft.

20. Verfahren nach Anspruch 19,
**dadurch gekennzeichnet,**
**dass** es das Bebrüten des sich aus dem Schritt (b) ergebenden Mediums bei etwa 4°C in Gegenwart von Clusterin aufweist.

21. Isolierte, oligomere Struktur aus einem lösbaren, kugelförmigen, nicht faserigen β-Amyloid, die nach Anspruch 19 oder 20 präpariert ist.

22. Verwendung der oligomeren Struktur nach einem der Ansprüche 1 bis 8 für die Änderung der Langzeitwirkungsreaktion einer Nervenzelle, wobei das Zusammenbringen der Zelle mit der oligomeren Struktur in vitro stattfindet.

23. Verwendung der oligomeren Struktur nach einem der Ansprüche 1 bis 8 für das Bewirken einer morphologischen Änderung einer Nervenzelle, wobei das Zusammenbringen der Zelle mit der oligomeren Struktur in vitro stattfindet.

24. Verwendung nach Anspruch 23,
**dadurch gekennzeichnet,**
**dass** die morphologische Änderung eine Wirkung umfasst, die aus der Gruppe ausgewählt ist, die aus der Zellabtötung, der Änderung der Fyn-Kinase-Aktivität, der Änderung des Fyn-Kinase-Subzellorts und der Änderung des mRNA-Pegels für Proteine besteht, die Interleukin-1, induzierbare Stickoxidsynthase, Apo E, Apo J und α1-Antichymotrypsin umfassen.

25. Verwendung der oligomeren Struktur nach einem der Ansprüche 1 bis 8 für das Bewirken einer astrozytischen Aktivierung, wobei das Zusammenbringen eines Astrozyten mit der oligomeren Struktur in vitro stattfindet.

26. Verwendung der oligomeren Struktur nach einem der Ansprüche 1 bis 8 zur Identifizierung der Testverbindungen, die die Nervenschädigung der oligomeren Struktur vermeiden, wobei das Zusammenbringen einer Nervenzelle mit der oligomeren Struktur und der Testverbindung in vitro stattfindet.

27. Verwendung der oligomeren Struktur nach einem der Ansprüche 1 bis 8 zur Identifizierung der Testverbindungen, die die Bindung an ein Zelloberflächenprotein der oligomeren Struktur verhindern, wobei das Zusammenbringen einer Nervenzelle mit der oligomeren Struktur und der Testverbindung in vitro stattfindet.

28. Verwendung der oligomeren Struktur nach einem der Ansprüche 1 bis 8 zur Identifizierung der Testverbindungen, die die Bildung der oligomeren Struktur verhindern, wobei das Zusammenbringen des Amyloid-β1-42-Proteins mit der Testverbindung während der Bebrütung zur Bildung der oligomeren Struktur stattfindet.

29. Verwendung der oligomeren Struktur nach einem der Ansprüche 1 bis 8 zum Bewirken einer ADDL-induzierten, fehlerbehafteten, neuronalen Signalisierung einer Nervenzelle, wobei das Zusammenbringen der Zelle mit der oligomeren Struktur stattfindet.

## Revendications

1. Structure oligomère amyloïde non fibrillaire, globulaire, soluble, homogène et isolée comprenant 4 à 12 protéines amyloïdes β1-42, dans laquelle la structure montre une neurotoxicité et ne contient pas de protéine amyloïde β1-40.

2. Structure oligomère selon la revendication 1, dans laquelle la structure oligomère comprend une forme oligomère choisie parmi le groupe constitué d'agrégats tétramères, pentamères et hexamères de protéine amyloïde β1-42.

3. Structure oligomère selon la revendication 1 ou 2, dans laquelle la structure oligomère a un poids moléculaire de 26 kD à 28 kD tel que déterminé par électrophorèse sur gel non dénaturant.

4. Structure oligomère selon une quelconque des revendications 1 à 3, dans laquelle la structure oligomère a un poids moléculaire de 22 kD à 24 kD ou de 18 kD à 19 kD tel que déterminé par électrophorèse sur gel de SDS-polyacrylamide 15%.

5. Structure oligomère selon une quelconque des revendications 1 à 4, dans laquelle la structure oligomère comprend des globules ayant des dimensions de 4,7 nm à 6,2 nm de la manière mesurée par microscopie à force atomique.

6. Structure oligomère selon une quelconque des revendications 1 à 5, dans laquelle la structure oligomère comprend des globules ayant des dimensions de 4,9 nm à 5,4 nm de la manière mesurée par microscopie à force atomique.

7. Structure oligomère selon une quelconque des revendications 1 à 5, dans laquelle la structure oligomère comprend des globules ayant des dimensions de 5,7 nm à 6,2 nm de la manière mesurée par microscopie à force atomique.

8. Structure oligomère selon une quelconque des revendications 1 à 5, dans laquelle de 40% à 75% de la structure oligomère comprend des globules ayant des dimensions de 4,9 nm à 5,4 nm, et des dimensions de 5,7 nm à 6,2 nm, de la manière mesurée par microscopie à force atomique.

9. Procédé pour identifier des composés d'essai qui bloquent la neurotoxicité de la structure oligomère d'une quelconque des revendications 1 à 8, le procédé consistant à ;
(a) mettre en contact des cultures séparées de cellules neuronales avec la structure oligomère, soit en présence ou en l'absence de contact avec le composé d'essai ;
(b) mesurer la proportion de cellules viables dans chaque culture ; et
(c) comparer la proportion de cellules viables dans chaque culture avec des composés qui bloquent la neurotoxicité de la structure oligomère qui est identifiée comme entraînant une proportion augmentée de cellules viables dans ladite culture comparativement à la culture correspondante mise en contact avec la structure oligomère en l'absence du composé d'essai.

10. Procédé pour identifier des composés d'essai qui bloquent la liaison à une protéine de surface cellulaire de la structure oligomère d'une quelconque des revendications 1 à 8, le procédé consistant à :
(a) mettre en contact des cultures séparées de cellules neuronales avec la structure oligomère soit en présence ou en l'absence de contact avec le composé d'essai ;
(b) ajouter un réactif qui se lie à la structure oligomère, le réactif étant fluorescent ;
(c) analyser lesdites cultures cellulaires séparées par trieur cellulaire à fluorescence ; et
(d) comparer la fluorescence des cultures avec des composés qui bloquent la liaison à une protéine de surface cellulaire de la structure oligomère qui est identifiée comme entraînant une fluorescence réduite de la culture comparativement à la culture correspondante mise en contact avec la structure oligomère en l'absence du composé d'essai.

11. Procédé pour identifier des composés qui bloquent la liaison à une protéine de surface cellulaire de la structure oligomère d'une quelconque des revendications 1 à 8, le procédé consistant à :
(a) former la structure oligomère à partir de protéine amyloïde β1-42 de telle sorte qu'elle devienne une structure oligomère marquée comprenant un fragment de liaison capable de se lier à un réactif fluorescent ;
(b) mettre en contact des cultures séparées de cellules neuronales avec la structure oligomère marquée soit en présence ou en l'absence de contact avec le composé d'essai ;
(c) ajouter un réactif fluorescent qui se lie à la structure oligomère ;
(d) analyser les cultures cellulaires séparées par un trieur cellulaire à fluorescence ; et
(e) comparer la fluorescence des cultures avec des composés qui bloquent la liaison à une protéine de surface cellulaire de la structure oligomère qui est identifiée comme entraînant une fluorescence réduite de la culture comparativement à la culture correspondante mise en contact avec la structure oligomère en l'absence du composé d'essai.

12. Procédé pour identifier des composés qui bloquent la formation ou la liaison à une protéine de surface cellulaire de la structure oligomère d'une quelconque des revendications 1 à 8, le procédé consistant à :
(a) préparer des échantillons séparés de protéine amyloïde β1-42 qui ont été ou n'ont pas été mélangées avec le composé d'essai ;
(b) former la structure oligomère dans les échantillons séparés ;
(c) mettre en contact des cultures séparées de cellules neuronales avec les échantillons séparés ;
(d) ajouter un réactif qui se lie à la structure oligomère, le réactif étant fluorescent ;
(e) analyser les cultures cellulaires séparées par un trieur cellulaire à fluorescence ; et
(f) comparer la fluorescence des cultures avec des composés qui bloquent la formation ou la liaison à une protéine de surface cellulaire de la structure oligomère qui est identifiée comme entraînant une fluorescence réduite de la culture comparativement à la culture correspondante mise en contact avec la structure oligomère en l'absence du composé d'essai.

13. Procédé pour identifier des composés qui bloquent la formation ou la liaison à une protéine de surface cellulaire de la structure oligomère d'une quelconque des revendications 1 à 8, le procédé consistant à :
(a) préparer des échantillons séparés de protéine amyloïde β1-42 qui ont été ou n'ont pas été mélangées avec le composé d'essai ;
(b) former la structure oligomère dans les échantillons séparés de telle sorte qu'elles deviennent une structure oligomère marquée comprenant un fragment de liaison capable de se lier à un réactif fluorescent dans chacun des échantillons séparés ;
(c) mettre en contact des cultures séparées de cellules neuronales avec les échantillons séparés ;
(d) ajouter un réactif fluorescent qui se lie à la structure oligomère ;
(e) analyser les cultures cellulaires séparées par un trieur cellulaire à fluorescence ; et
(f) comparer la fluorescence des cultures avec des composés qui bloquent la formation ou la liaison à une protéine de surface cellulaire de la structure oligomère qui est identifiée comme entraînant une fluorescence réduite de la culture comparativement à la culture correspondante mise en contact avec la structure oligomère en l'absence du composé d'essai.

14. Procédé de la revendication 12 ou 13, dans lequel la fluorescence des cultures est de plus comparée avec la fluorescence des cultures qui ont été traitées de la même façon à l'exception que, à la place d'ajouter ou de ne pas ajouter un composé d'essai avant la formation de la structure oligomère, le composé d'essai est soit ajouté ou n'est pas ajouté après la formation de la structure oligomère,
avec des composés qui bloquent la formation de la structure oligomère qui est identifiée comme entraînant une fluorescence réduite de la culture comparativement à la culture correspondante mise en contact avec la structure oligomère en l'absence du composé d'essai, seulement quand le composé est ajouté avant la structure oligomère, et
des composés qui bloquent la liaison à une protéine de surface cellulaire de la structure oligomère qui est identifiée comme entraînant une fluorescence réduite de la culture comparativement à la culture correspondante mise en contact avec la structure oligomère en l'absence du composé d'essai, quant le composé est ajouté soit avant ou après la structure oligomère.

15. Procédé de détection de liaison à une protéine de surface cellulaire de la structure oligomère d'une quelconque des reven-dications 1 à 8, le procédé consistant à ;
(a) former la structure oligomère à partir de protéine amyloïde β1-42 ;
(b) mettre en contact une culture de cellules neuronales avec la structure oligomère ;
(c) ajouter un anticorps qui se lie à ladite structure oligomère, l'anticorps incluant un fragment se conjuguant ;
(d) enlever l'anticorps non lié ;
(f) lier une enzyme à l'anticorps lié à la structure oligomère par l'intermédiaire du fragment se conjuguant ;
(g) ajouter un substrat incolore qui est clivé par l'enzyme pour donner un changement de couleur ; et
(h) déterminer le changement de couleur comme mesure de liaison à une protéine de surface cellulaire de la structure oligomère.

16. Procédé pour identifier des composés qui bloquent la liaison à une protéine de surface cellulaire de la structure oligomère d'une quelconque des revendications 1 à 8, le procédé consistant à :
(a) préparer des échantillons séparés de protéine β1-42 qui ont ou n'ont pas été mélangés avec le composé d'essai ;
(b) former la structure oligomère dans les échantillons séparés ;
(c) mettre en contact des cultures séparées de cellules neuronales avec les échantillons séparés ;
(d) ajouter un anticorps qui se lie à la structure oligomère, l'anticorps incluant un fragment se conjuguant ;
(e) enlever l'anticorps non lié ;
(f) lier une enzyme à l'anticorps lié à la structure oligomère par l'intermédiaire dudit fragment se conjuguant ;
(g) ajouter un substrat incolore qui est clivé par l'enzyme pour donner un changement de couleur ; et
(h) comparer le changement de couleur produit par chacun des échantillons séparés, avec des composés qui bloquent la formation ou la liaison à une protéine de surface cellulaire de la structure oligomère qui est identifiée comme entraînant un changement de couleur réduit produit par la culture comparativement à la culture correspondante mise en contact avec la structure oligomère en l'absence du composé d'essai.

17. Procédé de la revendication 16, dans lequel le changement de couleur produit par les cultures est de plus comparé avec le changement de couleur produit par des cultures qui ont été traitées de la même façon à l'exception que, à la place d'ajouter ou de ne pas ajouter un composé d'essai avant la formation de la structure oligomère, le (ledit) composé d'essai est soit ou n'est pas ajouté après la formation de la structure oligomère,
avec des composés qui bloquent la formation de la structure oligomère qui est identifiée comme entraînant un changement de couleur réduit produit par la culture comparativement à la culture correspondante mise en contact avec la structure oligomère en l'absence du composé d'essai, seulement quand le composé est ajouté avant la structure oligomère, et
des composés qui bloquent la liaison au récepteur de la structure oligomère qui est identifiée comme entraînant un changement de couleur réduit produit par la culture comparativement à la culture correspondante mise en contact avec la structure oligomère en l'absence du composé d'essai, quand le composé est ajouté soit avant ou après la structure oligomère.

18. Procédé pour identifier des composés qui bloquent la formation de la structure oligomère d'une quelconque des revendications 1 à 8, le procédé consistant à :
(a) préparer des échantillons séparés de protéine amyloïde β1-42 qui ont ou n'ont pas été mélangées avec le composé d'essai ;
(b) former la structure oligomère dans les échantillons séparés ;
(c) évaluer si de quelconques ensembles de protéine se sont formés dans les échantillons séparés en utilisant un procédé choisi parmi le groupe constitué de l'électrophorèse, l'immunoreconnaissance et la microscopie à force atomique ; et
(d) comparer la formation des ensembles de protéine dans les échantillons séparés, avec des composés qui bloquent la formation de la structure oligomère qui est identifiée comme entraînant une formation diminuée de la structure oligomère dans l'échantillon comparativement à un échantillon dans lequel la structure oligomère est formée en l'absence du composé d'essai.

19. Procédé pour préparer la structure oligomère selon une quelconque des revendications 1 à 8, dans lequel le procédé consiste à :
(a) obtenir une solution de protéine amyloïde β1-42 monomère, ladite protéine amyloïde β1-42 pouvant former la structure oligomère ;
(b) diluer la solution protéique dans un milieu approprié à une concentration d'environ 5 nM à environ 500 µM ;
(c) incuber le milieu résultant de l'étape (b) à environ 4°C pendant environ 2 heures à environ 48 heures ;
(d) centrifuger la solution à environ 14 000 g à environ 4°C ; et
(e) récupérer le surnageant provenant de la centrifugation comme contenant la structure oligomère amyloïde β1-42.

20. Procédé de la revendication 19, dans lequel le procédé consiste à incuber le milieu résultant de l'étape (b) à environ 4°C en présence de clustérine.

21. Structure oligomère amyloïde β non fibrillaire, globulaire, soluble, isolée, préparée selon la revendication 19 ou 20.

22. Utilisation de la structure oligomère selon une quelconque des revendications 1 à 8 pour modifier la réponse de potentialisation à long terme d'une cellule nerveuse, consistant à mettre en contact la cellule in vitro avec la structure oligomère.

23. Utilisation de la structure oligomère selon une quelconque des revendications 1 à 8 pour provoquer le changement morphologique d'une cellule nerveuse, consistant à mettre en contact la cellule in vitro avec la structure oligomère.

24. Utilisation selon la revendication 23, dans laquelle le changement morphologique inclut un effet choisi parmi le groupe constitué de la destruction de cellules, modification de l'activité de kinase Fyn, modification de la localisation sous-cellulaire de kinase Fyn et modification du taux d'ARNm pour des protéines comprenant l'interleukine-1, la synthase d'oxyde nitrique inductible, l'Apo E, l'Apo J et l'α1-antichymotrypsine.

25. Utilisation de la structure oligomère selon une quelconque des revendications 1 à 8 pour provoquer l'activation d'astrocytes, consistant à mettre en contact un astrocyte in vitro avec la structure oligomère.

26. Utilisation de la structure oligomère selon une quelconque des revendications 1 à 8 pour identifier des composés d'essai qui bloquent la neurotoxicité de la structure oligomère, consistant à mettre en contact une cellule nerveuse in vitro avec la structure oligomère et le composé d'essai.

27. Utilisation de la structure oligomère selon une quelconque des revendications 1 à 8 pour identifier des composés d'essai qui bloquent la liaison à une protéine de surface cellulaire de la structure oligomère, consistant à mettre en contact une cellule nerveuse in vitro avec la cellule oligomère et le composé d'essai.

28. Utilisation de la structure oligomère selon une quelconque des revendications 1 à 8 pour identifier des composés d'essai qui bloquent la formation de la structure oligomère, consistant à mettre en contact une protéine amyloïde β1-42 avec le composé d'essai durant l'incubation pour former la structure oligomère.

29. Utilisation de la structure oligomère selon une quelconque des revendications 1 à 8 pour provoquer le signal neuronal anormal induit par ADDL d'une cellule nerveuse, consistant à mettre en contact la cellule avec la structure oligomère.


### SEQUENCE LISTING

(1) GENERAL INFORMATION:
   (i) APPLICANT:
      (A) NAME: Acumen Pharmaceuticals, Inc. et al.
   (ii) TITLE OF INVENTION: Amyloid β Protein (Globular Assembly and Uses Thereof)
   (iii) NUMBER OF SEQUENCES: 4
   (iv) CORRESPONDENCE ADDRESS:
      (A) ADDRESSEE: McDonnell Boehnen Hulbert & Berghoff
      (B) STREET: 300 South Wacker Drive
      (C) CITY: Chicago
      (D) STATE: IL
      (E) COUNTRY: USA
      (F) ZIP: 60606
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (US)
   (vi) CURRENT APPLICATION DATA:
      (A) APPLICATION NUMBER: PCT/US
      (B) FILING DATE: 05-FEB-1998
      (C) CLASSIFICATION DATA:
   (vii) PRIOR APPLICATION DATA:
      (A) APPLICATION NUMBER: US 08/796,089
      (B) FILING DATE: 05-FEB-1997
(2) INFORMATION FOR SEQ ID NO: 1:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO: 1:
      GCACCTTCTT TCCCTTCATC 20
(2) INFORMATION FOR SEQ ID NO: 2:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO: 2:
      TGCTGATGTA CCAGTTGGGG 20
(2) INFORMATION FOR SEQ ID NO: 3:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO: 3:
      CAGTCCTTGA CCTGCGACC 19
(2) INFORMATION FOR SEQ ID NO: 4:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO: 4:
      GCCTCACATC ACATCCTTG 19